# EUROPEAN PATENT APPLICATION

(11) **EP 3 318 638 A1**
(43) Date of publication of application: **09.05.2018**
(21) Application number: 16382513.6
(22) Date of filing: 07.11.2016
(51) Int. Cl.: C12N 15/82, C07K 14/415

(54) **NUCLEOTIDE SEQUENCE FOR IMPROVING RESISTANCE AGAINST PLANT PATHOGENS**

(71) Applicant: CONSEJO SUPERIOR DE INVESTIGACIONES CIENTIFICAS, 28006 Madrid (ES)
(72) Inventor: GIMENEZ IBAÑEZ, Selena, 28006 Madrid (ES); SOLANO TAVIRA, Roberto, 28006 Madrid (ES); BOTER GIL, Marta, 28006 Madrid (ES); CHINI, Andrea, 28006 Madrid (IT); GARCÍA CASADO, Gloria, 28006 Madrid (ES)
(74) Representative: Pons Ariño, Angel

(57) **Abstract**

The invention relates to a nucleic acid sequence that improves resistance to biotrophic pathogens, in particular to *Pseudomonas syringae,* without affecting susceptibility to necrotrophs, in particular *Botrytis cinerea.* The present invention also relates to a plant that comprises the nucleic acid of the invention and a method to generate plants resistant to biotrophic pathogens and necrotrophs.

SEQUENCE LISTING

<110>  Consejo Superior de Investigaciones Científicas

<120>  Recombinant nucleotide sequence for improving resistance against
       plant pathogens

<130>  EP1641.1104_BIS

<160>  61

<170>  PatentIn version 3.5

<210>  1
<211>  750
<212>  DNA
<213>  Arabidopsis thaliana

<400>  1
atgtcgagtt tttctgccga gtgttgggac ttctctggtc gtaaaccgag cttttcacaa      60

acatgtactc gattgagtcg ttacctgaag gagaagggta gttttggaga tctgagctta     120

gggatgacat gcaagcccga cgttaatgga ggttcacgtc agcctacaat gatgaatctg     180

ttcccttgtg aagcttcagg aatggattct tctgctggtc aagaagacat taaaccgaag     240

actatgtttc cgagacaatc aagcttttct tcttcctctt cctctgggac caaagaagat     300

gtacagatga tcaaagagac tactaaatct gtgaagccag agtctcaatc tgctccgttg     360

actatattct acggtggtcg agttatggtg tttgatgatt tttctgctga gaaagctaaa     420

gaagtcattg atttggctaa caaaggaagt gccaaaagct tcacatgttt cacagctgaa     480

gtaaacaata accatagtgc ttattctcaa aaagagattg cttctagccc aaatcctgtt     540

tgtagtcctg caaaaaccgc agcacaagag ccaattcagc ctaacccggc ctctttagcc     600

tgcgaactcc cgattgcaag aagagcttca cttcatcggt tccttgagaa gaggaaggat     660

aggatcacat caaaggcacc ataccaaata gacggttcag ctgaagcgtc ttccaagcct     720

actaacccag cttggctcag ttcacggtaa                                      750

<210>  2
<211>  1340
<212>  DNA
<213>  Arabidopsis thaliana

<400>  2
   atgtcgagtt tttctgccga gtgttgggac ttctctggtc gtaaaccgag cttttcacaa      60
   acatgtactc gattgagtcg ttacctgaag gagaagggta gttttggaga tctgagctta     120
   gggatgacat gcaagcccga cgttaatggt aatcaacctt tctcttttag atctgttcat     180
   ttcgtttctg cgaattttga gtttcctgtt gcgaattacg aattatgaat cgattacatg     240
   ttgtctggaa attgaatcgt tgtagagaat cgatgattaa tggattttga atcaatttct     300
   atatatgtat gtatgtgtat gaatttgtgg agatggttct gtgtttggtt taagcataat     360
   cctctaaagc cataggcctg gtttagtcat taagtagatt ttgttttgca gttgtttgag 420
   agattggtga atgataataa tctttacttt tggatcaatt ttcgcaggag gttcacgtca 480
   gcctacaatg atgaatctgt tcccttgtga agcttcagga atggattctt ctgctggtca 540
   agaagacatt aaaccgaaga ctatgtttcc gagacaatca agcttttctt cttcctcttc 600
   ctctgggacc aaagaagatg tacagatgat caaagagact actaagtatg tgtttatatg 660
   tatatagtga aaagaatcgt ctcatatagt aagctatgtt tcatcagttt aactaatttt 720
   tgtgtgattt tgaacagatc tgtgaagcca gagtctcaat ctgctccgtt gactatattc 780
   tacggtggtc gagttatggt gtttgatgat ttttctgctg agaaagctaa agaagtcatt 840
   gatttggcta acaaaggaag tgccaaaagc ttcacatgtt tcacagctga agtaaacaat 900
   aaccatagtg cttattctca aaaagagatt gcttctagcc caaatcctgt ttgtagtcct 960
   gcaaaaaccg cagcacaaga gccaattcag cctaacccgg cctctttagc ctgcggtaat 1020
   tgatctcagc ctcttttggt tcaaccatct ctgagcttgg cttagtccta tttctctaac 1080
   tttctctctt tttgatttgt agaactcccg attgcaagaa gagcttcact tcatcggttc 1140
   cttgagaaga ggaaggatag gtaatgcatt ttaatgaaaa cataatatct tgttattcca 1200
   taagttcttg ttaagcgaat ttctcaatat cctactcgta aacctttaac aggatcacat 1260
   caaaggcacc ataccaaata gacggttcag ctgaagcgtc ttccaagcct actaacccag 1320
   cttggctcag ttcacggtaa 1340

<210> 3
<211> 249
<212> PRT
<213> Arabidopsis thaliana

<400> 3

Met Ser Ser Phe Ser Ala Glu Cys Trp Asp Phe Ser Gly Arg Lys Pro
1               5                   10                  15

Ser Phe Ser Gln Thr Cys Thr Arg Leu Ser Arg Tyr Leu Lys Glu Lys
            20                  25                  30

Gly Ser Phe Gly Asp Leu Ser Leu Gly Met Thr Cys Lys Pro Asp Val
        35                  40                  45

Asn Gly Gly Ser Arg Gln Pro Thr Met Met Asn Leu Phe Pro Cys Glu
    50                  55                  60

Ala Ser Gly Met Asp Ser Ser Ala Gly Gln Glu Asp Ile Lys Pro Lys
65                  70                  75                  80

Thr Met Phe Pro Arg Gln Ser Ser Phe Ser Ser Ser Ser Ser Ser Gly
                85                  90                  95

Thr Lys Glu Asp Val Gln Met Ile Lys Glu Thr Thr Lys Ser Val Lys
            100                 105                 110

Pro Glu Ser Gln Ser Ala Pro Leu Thr Ile Phe Tyr Gly Gly Arg Val
        115                 120                 125

Met Val Phe Asp Asp Phe Ser Ala Glu Lys Ala Lys Glu Val Ile Asp
    130                 135                 140

Leu Ala Asn Lys Gly Ser Ala Lys Ser Phe Thr Cys Phe Thr Ala Glu
145                 150                 155                 160

Val Asn Asn Asn His Ser Ala Tyr Ser Gln Lys Glu Ile Ala Ser Ser
                165                 170                 175

Pro Asn Pro Val Cys Ser Pro Ala Lys Thr Ala Ala Gln Glu Pro Ile
            180                 185                 190

Gln Pro Asn Pro Ala Ser Leu Ala Cys Glu Leu Pro Ile Ala Arg Arg
        195                 200                 205

Ala Ser Leu His Arg Phe Leu Glu Lys Arg Lys Asp Arg Ile Thr Ser
    210                 215                 220

Lys Ala Pro Tyr Gln Ile Asp Gly Ser Ala Glu Ala Ser Ser Lys Pro
225                 230                 235                 240

Thr Asn Pro Ala Trp Leu Ser Ser Arg
                245

<210>  4
<211>  28
<212>  PRT
<213>  Artificial Sequence

<220>
<223>  Consensus ZIM domain


<220>
<221>  misc_feature
<222>  (1)..(1)
<223>  Gln, Pro, Arg or Ile

<220>
<221>  misc_feature
<222>  (2)..(2)
<223>  Leu, Met or Ile

<220>
<221>  misc_feature
<222>  (5)..(5)
<223>  Phe or Ile

<220>
<221>  misc_feature
<222>  (6)..(6)
<223>  Tyr or Phe

<220>
<221>  misc_feature
<222>  (7)..(7)
<223>  Gly, Ala or Asn

<220>
<221>  misc_feature
<222>  (9)..(9)
<223>  Ser, Lys, Arg, Thr, His or Gln

<220>
<221>  misc_feature
<222>  (10)..(10)
<223>  Val, Met, Phe or Ile

<220>
<221>  misc_feature
<222>  (11)..(11)
<223>  Cys, Ile, Leu, Met, Thr or Ser

<220>
<221>  misc_feature
<222>  (12)..(12)
<223>  Val or Phe

<220>
<221>  misc_feature
<222>  (13)..(13)
<223>  Phe, Tyr or Ser

<220>
<221>  misc_feature
<222>  (14)..(14)
<223>  Asp, Asn, Ser or Gln

<220>
<221>  misc_feature
<222>  (15)..(15)
<223>  Asp, Glu, Gly or Val

<220>
<221>  misc_feature
<222>  (16)..(16)
<223>  Phe, Ile, Leu, Val or Ser

<220>
<221>  misc_feature
<222>  (17)..(17)
<223>  Pro, Ser, Thr or Ala

<220>
<221>  misc_feature
<222>  (18)..(18)

<223>  Ala, Pro, Arg, His, Val, Glu or Ser

<220>
<221>  misc_feature
<222>  (19)..(19)
<223>  Glu, Asp, Leu or Asn

<220>
<221>  misc_feature
<222>  (20)..(20)
<223>  Lys, Glu or Gln

<220>
<221>  misc_feature
<222>  (21)..(21)
<223>  Ala or Val

<220>
<221>  misc_feature
<222>  (22)..(22)
<223>  Lys, Gln, Gly or Asn

<220>
<221>  misc_feature
<222>  (23)..(23)
<223>  Glu, Ala or Ser

<220>
<221>  misc_feature
<222>  (24)..(24)
<223>  Ile or Val

<220>
<221>  misc_feature
<222>  (25)..(25)
<223>  Met, Leu or Ile

<220>
<221>  misc_feature
<222>  (26)..(26)
<223>  Leu, Ser, Asn, Asp, Glu, Arg, His or Lys

<220>
<221>  misc_feature
<222>  (27)..(27)
<223>  Leu, Ile, Val or Cys

<400>  4

Xaa Xaa Thr Ile Xaa Xaa Xaa Gly Xaa Xaa Xaa Xaa Xaa Xaa Xaa Xaa
1               5                   10                  15

Xaa Xaa Xaa Xaa Xaa Xaa Xaa Xaa Xaa Xaa Xaa Ala
            20                  25

<210>  5
<211>  28
<212>  PRT
<213>  Arabidopsis thaliana

<400>  5

Pro Leu Thr Ile Phe Tyr Gly Gly Arg Val Met Val Phe Asp Asp Phe
1               5                   10                  15

Ser Ala Glu Lys Ala Lys Glu Val Ile Asp Leu Ala
            20                  25

<210>  6
<211>  27
<212>  PRT
<213>  Artificial Sequence

<220>
<223>  Consensus sequence for Jas domain in Arabidopsis

<220>
<221>  MISC_FEATURE
<222>  (1)..(1)
<223>  Asp, Glu, Val, Ser or Ala

<220>
<221>  MISC_FEATURE
<222>  (2)..(2)
<223>  Leu, Val, Glu or Lys

<220>
<221>  MISC_FEATURE
<222>  (3)..(3)
<223>  Pro, Arg or Ala

<220>
<221>  MISC_FEATURE
<222>  (4)..(4)
<223>  Ile, Gln, Ser or Leu

<220>
<221>  MISC_FEATURE
<222>  (5)..(5)
<223>  Ala, Met or Thr

<220>
<221>  MISC_FEATURE
<222>  (6)..(6)
<223>  Arg or Lys

<220>
<221>  MISC_FEATURE
<222>  (7)..(7)
<223>  Arg or Lys

<220>
<221>  MISC_FEATURE
<222>  (8)..(8)
<223>  Ala, Ser, His, Arg or Lys

<220>
<221>  misc_feature
<222>  (9)..(9)
<223>  Xaa can be any naturally occurring amino acid

<220>
<221>  MISC_FEATURE
<222>  (11)..(11)
<223>  Gln, His or Ala

<220>
<221>  MISC_FEATURE
<222>  (12)..(12)
<223>  Arg or Ser

<220>
<221>  MISC_FEATURE
<222>  (14)..(14)
<223>  Leu or Phe

<220>
<221>  MISC_FEATURE
<222>  (15)..(15)
<223>  Glu, Ala or Gln

<220>
<221>  MISC_FEATURE
<222>  (18)..(18)
<223>  Lys, Arg or Ser

<220>
<221>  MISC_FEATURE
<222>  (19)..(19)
<223>  Asp, Glu, Leu, Ile or His

<220>
<221>  MISC_FEATURE
<222>  (21)..(21)
<223>  Leu, Ile, Val, Ala or Phe

<220>
<221>  MISC_FEATURE
<222>  (22)..(22)
<223>  Val, Thr, Ile, Met or Gln

<220>
<221>  MISC_FEATURE
<222>  (23)..(23)
<223>  ser, Ala, Asn or His

<220>
<221>  MISC_FEATURE
<222>  (24)..(24)
<223>  Thr, Lys, Arg, Val or Ala

<220>
<221>  MISC_FEATURE
<222>  (25)..(25)
<223>  Ser, Ala, Asn, Lys or Met

<400>  6

Xaa Xaa Xaa Xaa Xaa Xaa Xaa Xaa Xaa Leu Xaa Xaa Phe Xaa Xaa Lys
1               5                   10                  15

Arg Xaa Xaa Arg Xaa Xaa Xaa Xaa Xaa Pro Tyr
            20                  25

<210>  7
<211>  27
<212>  PRT
<213>  Arabidopsis thaliana

<400>  7

Glu Leu Pro Ile Ala Arg Arg Ala Ser Leu His Arg Phe Leu Glu Lys
1               5                   10                  15

Arg Lys Asp Arg Ile Thr Ser Lys Ala Pro Tyr
            20                  25

<210>  8
<211>  477
<212>  DNA
<213>  Artificial Sequence

<220>
<223>  JAZ2DeltaJas cDNA

<400>  8
atgtcgagtt tttctgccga gtgttgggac ttctctggtc gtaaaccgag cttttcacaa      60

acatgtactc gattgagtcg ttacctgaag gagaagggta gttttggaga tctgagctta     120

gggatgacat gcaagcccga cgttaatgga ggttcacgtc agcctacaat gatgaatctg     180

ttcccttgtg aagcttcagg aatggattct tctgctggtc aagaagacat taaaccgaag     240

actatgtttc cgagacaatc aagcttttct tcttcctctt cctctgggac caaagaagat     300

gtacagatga tcaaagagac tactaaatct gtgaagccag agtctcaatc tgctccgttg     360

actatattct acggtggtcg agttatggtg tttgatgatt tttctgctga gaaagctaaa     420

gaagtcattg atttggctaa caaaggaagt gccaaaagct tcacatgttt cacatga        477

<210>  9
<211>  888
<212>  DNA
<213>  Artificial Sequence

<220>
<223>  Jaz2DeltaJas gDNA

<400>  9
atgtcgagtt tttctgccga gtgttgggac ttctctggtc gtaaaccgag cttttcacaa      60

acatgtactc gattgagtcg ttacctgaag gagaagggta gttttggaga tctgagctta     120

gggatgacat gcaagcccga cgttaatggt aatcaacctt tctcttttag atctgttcat     180

ttcgtttctg cgaattttga gtttcctgtt gcgaattacg aattatgaat cgattacatg     240

ttgtctggaa attgaatcgt tgtagagaat cgatgattaa tggattttga atcaatttct     300

atatatgtat gtatgtgtat gaatttgtgg agatggttct gtgtttggtt taagcataat     360

cctctaaagc cataggcctg gtttagtcat taagtagatt ttgttttgca gttgtttgag 420

agattggtga atgataataa tctttacttt tggatcaatt ttcgcaggag gttcacgtca 480

gcctacaatg atgaatctgt tcccttgtga agcttcagga atggattctt ctgctggtca 540

agaagacatt aaaccgaaga ctatgtttcc gagacaatca agcttttctt cttcctcttc 600

ctctgggacc aaagaagatg tacagatgat caaagagact actaagtatg tgtttatatg 660

tatatagtga aaagaatcgt ctcatatagt aagctatgtt tcatcagttt aactaatttt 720

tgtgtgattt tgaacagatc tgtgaagcca gagtctcaat ctgctccgtt gactatattc 780

tacggtggtc gagttatggt gtttgatgat ttttctgctg agaaagctaa agaagtcatt 840

gatttggcta acaaaggaag tgccaaaagc ttcacatgtt tcacatga 888

<210> 10
<211> 158
<212> PRT
<213> Artificial Sequence

<220>
<223> JAZ2DeltaJas aminoacids

<400> 10

Met Ser Ser Phe Ser Ala Glu Cys Trp Asp Phe Ser Gly Arg Lys Pro
1               5                   10                  15

Ser Phe Ser Gln Thr Cys Thr Arg Leu Ser Arg Tyr Leu Lys Glu Lys
            20                  25                  30

Gly Ser Phe Gly Asp Leu Ser Leu Gly Met Thr Cys Lys Pro Asp Val
        35                  40                  45

Asn Gly Gly Ser Arg Gln Pro Thr Met Met Asn Leu Phe Pro Cys Glu
    50                  55                  60

Ala Ser Gly Met Asp Ser Ser Ala Gly Gln Glu Asp Ile Lys Pro Lys
65                  70                  75                  80

Thr Met Phe Pro Arg Gln Ser Ser Phe Ser Ser Ser Ser Ser Ser Gly
                85                  90                  95

Thr Lys Glu Asp Val Gln Met Ile Lys Glu Thr Thr Lys Ser Val Lys
            100                 105                 110

Pro Glu Ser Gln Ser Ala Pro Leu Thr Ile Phe Tyr Gly Gly Arg Val
        115                 120                 125

Met Val Phe Asp Asp Phe Ser Ala Glu Lys Ala Lys Glu Val Ile Asp

130                 135                 140

Leu Ala Asn Lys Gly Ser Ala Lys Ser Phe Thr Cys Phe Thr
145                 150                 155

<210>  11
<211>  503
<212>  DNA
<213>  Arabidopsis thaliana

<400>  11
ctaaatggta taactgaaag actatcataa tcaaatttca tctgaacatt gatttgtttg      60

aactaattaa ttttaggatt ttacgttttc ataatttttt attttatttg aacatcttgt     120

aagggtaaaa tggtaaaaac aggagtagta agagggtgaa atggttattt caagtgtgaa     180

gcgtggggac gtgggtgacg gtggtgatct gaaacagtgg tctgccgtta aaacattaaa     240

ttaaaacacg tgttttaaat gtctctatgt taattaatac tataagagta tattttacta     300

atttccgtat ctgcaaccag cgaaaaaaaa gtaataaaga ggtcctccat ttcttcctcg     360

tgacaaaacg cacttggcag agaaagataa acaagaaccc taagtttttt tataagattc     420

gagaaaattc aacaactcag gaaggaagat ccttttgctc caatttctca atcgaaacga     480

tttcaatttc ggtttcaacg atg                                             503

<210>  12
<211>  31
<212>  DNA
<213>  Artificial Sequence

<220>
<223>  Jaz2.3 trasposon insertion mutant

<400>  12
atgtcgagtt tttctgccga gtgttgggac t                                     31

<210>  13
<211>  2021
<212>  DNA
<213>  Arabidopsis thaliana

<400>  13
gatctggctc gacgtcgacg agaagcagga tgagccatca aaggatccac taaagcacca      60

aactcaaaaa aaagagagcc aaatattgca actactcaag tcagtctaaa tatagttaaa     120

tgctgcgcta tatatcatgc atatacatat gtcatttgat tttctctttg ccattattta     180

ctctcattcg attttaatac aaaattattt aagttgttgt tgtaaacaga cgttgctgtt     240

gctgttgagt ttgatttaga atagttaagt ttggttttgg ttggagaaaa gttttcgttg     300

ttaagccaac tcgtgatcat ttaatctgtt cttttttggg tagggggtga tcattgaatc     360

ttttgttttg agttttgtat gtctatatag agtctaatac cattaaaaca aaaaaaatgt     420

cgtggtaacc agaattaaat gatgtaaaaa cagaaaacaa gaattaaatg tcggatgtaa 480

aaacagaatt aaatttcgga tgttttatgt ggatcatagc gtgagagatg cgtttatggc 540

atcttttgat ataatcttcg ctttgtaggt ccacttttga tctgatctga tctattatct 600

atatggaatg ttgtgtatat aataaactgc agtgtagtat tttcttaata atgtgatatt 660

aatatctaaa ttctaggctg gagaaaatga cgtgtgctgc atatccccca gactcgaaat 720

aacgatgtta tcgtgggaga tgatcgtcaa tttttttttt gtttagagat cgtcaagttg 780

ttgtgatcat tgactccttt ccattttttc tatagatttg aattttaaat attcattttc 840

ataaaaaaat gaaactttta ttattatatt tgtgagataa ttagatatgt tttaaaacat 900

tagtaaattt agtatagtat tagtttttac taaattaaga agaaagtgac gcacgaaatg 960

gcgtaatttc ctcccccgga ccacatgcat cttataaata cacaaaaacg atgtcaacga 1020

gtgattttag taatatgtac attaaagtaa tattttcttt ttggtaccaa taattacctt 1080

aattaataca agtatagttt aagcaacaag caacattctt taataattcg cgaaaaatat 1140

taataaaagt acaatgacag aaaaaataat taaaaattgg attaaaaaaa ataatattag 1200

agtgatgaca taatcacatg aggatttaga tttagcttaa taatgtttag ttgtggaact 1260

aaatttccgc aacaattatt ttgttgtatt taaatcattt tatgaaagtc taatctttac 1320

aattacttta tagtaccatc cgattaatca aagattaaaa atatagtact aaaatacaaa 1380

ttaatatatt ttgccaaaaa aatgcaacgt gcaaatgttt atcttggtca agagaacgtg 1440

cacgaaagta gtgtaactaa ttttactatt ttagattgag cggtgagaca aaaagggggt 1500

tagactcacg cgctacactt agcgcgtcag ccgacaaaaa aaagtgttgt cattgaataa 1560

aaaaataaat taaaactgcc gcttcgcatc tcgtgaatca ttcacgttaa aatagcgcac 1620

gtgcctcgcc acgcgtctca ccacaaaaag aaacgccaca cactgcacga cgatgcacgt 1680

gcctttttaa ccgactatat ccggtgacgt aaccgggaca acctgtagct ggtttttgcg 1740

ccaataggat atgagaaaaa agaatctttt ttttcttgtt atagttataa attaatggta 1800

cgattcacat gtcgcatgcg atttgttaat aaaactagaa attgcggtga attaacttca 1860

ttccacgttt tttcattttc tccctcaaaa gtctctgttt tttttccttt ttccggcgaa 1920

gctctattta gcttgattcc ggcgtttaac acgcgtttta atcgaaacag acatttgaga 1980

tcgaattaat tttgtagcgg gctgtgtctt tattatagat g 2021

<210> 14
<211> 894
<212> DNA
<213> Arabidopsis thaliana

<400> 14
atggagagag attttctcgg gttgggttcg aaaaattctc cgatcactgt caaggaggaa 60

accagcgaaa gctctagaga ttcagctccc aacagaggaa tgaactggtc tttctcaaac 120

aaagtatcag cttcttcttc tcagtttcta tccttcaggc caactcaaga agatagacat 180

agaaagtctg gaaattatca tcttcctcac tctggttcct tcatgccatc atcagtagct 240

gatgtttatg attcaacccg caaagctcct tacagttctg tacagggagt gaggatgttc 300

cctaattcca atcaacacga agaaactaac gcagtttcca tgtcgatgcc gggtttccag 360

tctcatcatt atgcaccagg aggaagaagc ttcatgaaca ataacaataa ctcacaacct 420

ttggtaggag ttcctatcat ggcacctcca atttcaatcc ttcctcctcc aggttccatt 480

gtagggacta ctgatattag atcttcttcc aagccaatag gttcacctgc gcagttgacg 540

atcttttatg ccggttcagt ttgtgtttac gatgacatat ctcctgaaaa ggcaaaggcg 600

ataatgttgc tagctgggaa cggttcctct atgcctcaag tcttttcgcc gcctcaaact 660

catcaacaag tggtccatca tactcgtgcc tctgtcgatt cttcagctat gcctcctagc 720

ttcatgccta caatatctta tcttagccct gaagctggaa gtagcacaaa cggactcgga 780

gccacaaaag cgacaagagg cttgacgtca acatatcaca acaaccaagc taatggatcc 840

aatattaact gcccagtacc agtttcttgt tctaccaatg taatggctcc aaca 894

<210> 15
<211> 312
<212> PRT
<213> Artificial Sequence

<220>
<223> JAZ3DeltaJas

<400> 15

Met Glu Arg Asp Phe Leu Gly Leu Gly Ser Lys Asn Ser Pro Ile Thr
1               5                   10                  15

Val Lys Glu Glu Thr Ser Glu Ser Ser Arg Asp Ser Ala Pro Asn Arg
            20                  25                  30

Gly Met Asn Trp Ser Phe Ser Asn Lys Val Ser Ala Ser Ser Ser Gln
        35                  40                  45

Phe Leu Ser Phe Arg Pro Thr Gln Glu Asp Arg His Arg Lys Ser Gly
    50                  55                  60

Asn Tyr His Leu Pro His Ser Gly Ser Phe Met Pro Ser Ser Val Ala
65                  70                  75                  80

Asp Val Tyr Asp Ser Thr Arg Lys Ala Pro Tyr Ser Ser Val Gln Gly
                85                  90                  95

Val Arg Met Phe Pro Asn Ser Asn Gln His Glu Glu Thr Asn Ala Val
            100                 105                 110

Ser Met Ser Met Pro Gly Phe Gln Ser His His Tyr Ala Pro Gly Gly
        115                 120                 125

Arg Ser Phe Met Asn Asn Asn Asn Asn Ser Gln Pro Leu Val Gly Val
    130                 135                 140

Pro Ile Met Ala Pro Pro Ile Ser Ile Leu Pro Pro Pro Gly Ser Ile
145                 150                 155                 160

Val Gly Thr Thr Asp Ile Arg Ser Ser Ser Lys Pro Ile Gly Ser Pro
                165                 170                 175

Ala Gln Leu Thr Ile Phe Tyr Ala Gly Ser Val Cys Val Tyr Asp Asp
            180                 185                 190

Ile Ser Pro Glu Lys Ala Lys Ala Ile Met Leu Leu Ala Gly Asn Gly
        195                 200                 205

Ser Ser Met Pro Gln Val Phe Ser Pro Pro Gln Thr His Gln Gln Val
    210                 215                 220

Val His His Thr Arg Ala Ser Val Asp Ser Ser Ala Met Pro Pro Ser
225                 230                 235                 240

Phe Met Pro Thr Ile Ser Tyr Leu Ser Pro Glu Ala Gly Ser Ser Thr
                245                 250                 255

Asn Gly Leu Gly Ala Thr Lys Ala Thr Arg Gly Leu Thr Ser Thr Tyr
            260                 265                 270

His Asn Asn Gln Ala Asn Gly Ser Asn Ile Asn Cys Pro Val Pro Val
        275                 280                 285

Ser Cys Ser Thr Asn Val Met Ala Pro Thr Gly Lys Lys Gln Ser Gln
    290                 295                 300

Arg Pro Asp Thr Thr Phe Ala Ile
305                 310

<210> 16
<211> 28
<212> PRT
<213> Arabidopsis thaliana

<400> 16

Pro Leu Thr Ile Phe Tyr Ala Gly Gln Val Ile Val Phe Asn Asp Phe
1               5                   10                  15

Ser Ala Glu Lys Ala Lys Glu Val Ile Asn Leu Ala
            20                  25

<210>  17
<211>  28
<212>  PRT
<213>  Arabidopsis thaliana

<400>  17

Gln Leu Thr Ile Phe Tyr Ala Gly Ser Val Cys Val Tyr Asp Asp Ile
1               5                   10                  15

Ser Pro Glu Lys Ala Lys Ala Ile Met Leu Leu Ala
            20                  25

<210>  18
<211>  28
<212>  PRT
<213>  Arabidopsis thaliana

<400>  18

Gln Leu Thr Ile Phe Tyr Ala Gly Ser Val Leu Val Tyr Gln Asp Ile
1               5                   10                  15

Ala Pro Glu Lys Ala Gln Ala Ile Met Leu Leu Ala
            20                  25

<210>  19
<211>  28
<212>  PRT
<213>  Arabidopsis thaliana

<400>  19

Gln Leu Thr Ile Phe Phe Gly Gly Lys Val Leu Val Tyr Asn Glu Phe
1               5                   10                  15

Pro Val Asp Lys Ala Lys Glu Ile Met Glu Val Ala
            20                  25

<210>  20
<211>  28
<212>  PRT
<213>  Arabidopsis thaliana

<400>  20

Gln Leu Thr Ile Phe Phe Gly Gly Lys Val Met Val Phe Asn Glu Phe
1               5                   10                  15

Pro Glu Asp Lys Ala Lys Glu Ile Met Glu Val Ala
            20                  25

<210>  21
<211>  28
<212>  PRT
<213>  Arabidopsis thaliana

<400>  21

Ile Leu Thr Ile Phe Tyr Asn Gly His Met Cys Val Ser Ser Asp Leu
1               5                   10                  15

Thr His Leu Glu Ala Asn Ala Ile Leu Ser Leu Ala
            20                  25

<210>  22
<211>  28
<212>  PRT
<213>  Arabidopsis thaliana

<400>  22

Arg Ile Thr Ile Phe Tyr Asn Gly Lys Met Cys Phe Ser Ser Asp Val
1               5                   10                  15

Thr His Leu Gln Ala Arg Ser Ile Thr Ser Ile Ala
            20                  25

<210>  23
<211>  28
<212>  PRT
<213>  Arabidopsis thaliana

<400>  23

Gln Leu Thr Ile Phe Tyr Gly Gly Thr Ile Ser Val Phe Asn Asp Ile
1               5                   10                  15

Ser Pro Asp Lys Ala Gln Ala Ile Met Leu Cys Ala
            20                  25

<210>  24
<211>  27
<212>  PRT
<213>  Arabidopsis thaliana

<400>  24

Pro Met Thr Ile Phe Tyr Asn Gly Ser Val Ser Val Phe Gln Val Ser
1               5                   10                  15

Arg Asn Lys Ala Gly Glu Ile Met Lys Val Ala

20                  25


<210>  25
<211>  28
<212>  PRT
<213>  Arabidopsis thaliana

<400>  25

Gln Leu Thr Ile Ile Phe Gly Gly Ser Phe Ser Val Phe Asp Gly Ile
1               5                   10                  15


Pro Ala Glu Lys Val Gln Glu Ile Leu His Ile Ala
            20                  25


<210>  26
<211>  28
<212>  PRT
<213>  Arabidopsis thaliana

<400>  26

Gln Leu Thr Ile Phe Phe Gly Gly Ser Val Thr Val Phe Asp Gly Leu
1               5                   10                  15


Pro Ser Glu Lys Val Gln Glu Ile Leu Arg Ile Ala
            20                  25


<210>  27
<211>  4
<212>  PRT
<213>  Artificial Sequence

<220>
<223>  Consensus TiFY


<220>
<221>  VARIANT
<222>  (3)..(3)
<223>  Thr or conserved amino adic

<220>
<221>  VARIANT
<222>  (4)..(4)
<223>  Tyr or conserved amino adic

<400>  27

Thr Ile Xaa Xaa
1


<210>  28
<211>  4
<212>  PRT
<213>  Artificial Sequence

<220>
<223>  TIFY domain

<400>  28

Thr Ile Phe Tyr
1


<210>  29
<211>  24
<212>  DNA
<213>  Artificial Sequence

<220>
<223>  forward primer pJAZ1 fw

<400>  29
caccgtacgt tccaacttct aacc                                              24


<210>  30
<211>  28
<212>  DNA
<213>  Artificial Sequence

<220>
<223>  reverse primer pJAZ1 rev

<400>  30
catctttaac aattaaaact ttcaaacg                                          28


<210>  31
<211>  25
<212>  DNA
<213>  Artificial Sequence

<220>
<223>  forward primer pJAZ2 fw

<400>  31
caccgactaa gaatttgtta tgaag                                             25


<210>  32
<211>  26
<212>  DNA
<213>  Artificial Sequence

<220>
<223>  reverse primer pJAZ2 rev

<400>  32
catcgttgaa accgaaattg aaatcg                                            26


<210>  33
<211>  30
<212>  DNA
<213>  Artificial Sequence

<220>

<223>  forward primer pJAZ3 fw

<400>  33
caccgatctg gctcgacgtc gacgagaagc                                        30


<210>  34
<211>  25
<212>  DNA
<213>  Artificial Sequence

<220>
<223>  reverse primer pJAZ3 rev

<400>  34
catctataat aaagacacag cccgc                                             25


<210>  35
<211>  16
<212>  DNA
<213>  Artificial Sequence

<220>
<223>  forward primer AtANAC19_fw

<400>  35
gcatctcgtc gctcag                                                       16


<210>  36
<211>  17
<212>  DNA
<213>  Artificial Sequence

<220>
<223>  reverse primer AtANAC19_rev

<400>  36
ctcgacttcc tcctccg                                                      17


<210>  37
<211>  16
<212>  DNA
<213>  Artificial Sequence

<220>
<223>  forward primer AtANAC55_fw

<400>  37
gcgctgcctc atagtc                                                       16


<210>  38
<211>  17
<212>  DNA
<213>  Artificial Sequence

<220>
<223>  reverse primer AtANAC55_rev

<400>  38

cgaggaatcc cctcagt                                                    17

<210>  39
<211>  17
<212>  DNA
<213>  Artificial Sequence

<220>
<223>  forward primer AtANAC72_fw

<400>  39
tgggtgttgt gtcgaat                                                    17

<210>  40
<211>  18
<212>  DNA
<213>  Artificial Sequence

<220>
<223>  reverse primer AtANAC72_rev

<400>  40
atcgtaacca ccgtaact                                                   18

<210>  41
<211>  21
<212>  DNA
<213>  Artificial Sequence

<220>
<223>  forward primer AtACTIN8_fw

<400>  41
ccagtggtcg tacaaccggt a                                               21

<210>  42
<211>  24
<212>  DNA
<213>  Artificial Sequence

<220>
<223>  reverse primer AtACTIN8_rev

<400>  42
tagttctttt cgatggagga gctg                                            24

<210>  43
<211>  20
<212>  DNA
<213>  Artificial Sequence

<220>
<223>  AtJAZ2 Full Length primer Forward (AtJAZ2 FL_F)

<400>  43
cgagtgttgg gacttctctg                                                 20

<210>  44
<211>  21
<212>  DNA
<213>  Artificial Sequence

<220>
<223>  AtJAZ2 Full Length Primer Reverse (AtJAZ2 FL_R)

<400>  44
gctcttcttg caatcgggag t                                                21


<210>  45
<211>  20
<212>  DNA
<213>  Artificial Sequence

<220>
<223>  AtJAZ2-3 Forward primer (AtJAZ2-3_F)

<400>  45
tggattcttc tgctggtcaa                                                  20


<210>  46
<211>  20
<212>  DNA
<213>  Artificial Sequence

<220>
<223>  AtJAZ2DeltaJas Forward primer (JAZ2DeltaJas_F)

<400>  46
tgcttctagc ccaaatcctg                                                  20


<210>  47
<211>  20
<212>  DNA
<213>  Artificial Sequence

<220>
<223>  AtJAZ2DeltaJas Reverse primer (JAZ2DeltaJas_R)

<400>  47
tctttggtcc cagaggaaga                                                  20


<210>  48
<211>  20
<212>  DNA
<213>  Artificial Sequence

<220>
<223>  AtPR1 Forward primer

<400>  48
gtgggttagc gagaaggcta                                                  20


<210>  49
<211>  20
<212>  DNA

<213>  Artificial Sequence

<220>
<223>  AtPR1 Reverse primer

<400>  49
actttggcac atccgagtct                                                   20


<210>  50
<211>  22
<212>  DNA
<213>  Artificial Sequence

<220>
<223>  AtPR2_Forward primer

<400>  50
gtctgaatca aggagcttag cc                                                22


<210>  51
<211>  21
<212>  DNA
<213>  Artificial Sequence

<220>
<223>  AtPR2 reverse primer

<400>  51
ccgtagcata ctccgatttg t                                                 21


<210>  52
<211>  21
<212>  DNA
<213>  Artificial Sequence

<220>
<223>  AtANAC19 G-box Forward primer

<400>  52
agcagctact acgagttgtg t                                                 21


<210>  53
<211>  20
<212>  DNA
<213>  Artificial Sequence

<220>
<223>  At ANAC19 G-box Reverse primer

<400>  53
cgtgtccacg tgtctatcgt                                                   20


<210>  54
<211>  20
<212>  DNA
<213>  Artificial Sequence

<220>

<223>  At ANAC55 G-box Forward primer

<400>  54
tgtgtcggct tgtggtagtt                                                    20


<210>  55
<211>  21
<212>  DNA
<213>  Artificial Sequence

<220>
<223>  At ANAC55 G-box Rreverse primer

<400>  55
gggatgagtt cactggatgg t                                                  21


<210>  56
<211>  20
<212>  DNA
<213>  Artificial Sequence

<220>
<223>  At ANAC72 G-box Forward primer

<400>  56
tgcaatcact cagcggactt                                                    20


<210>  57
<211>  20
<212>  DNA
<213>  Artificial Sequence

<220>
<223>  At ANAC72 G-box Rreverse primer

<400>  57
ggccgacctt atcgatgtgt                                                    20


<210>  58
<211>  25
<212>  DNA
<213>  Artificial Sequence

<220>
<223>  AtACTIN8 ChIP Forward primer

<400>  58
gactcagatc atgtttgaga ccttt                                              25


<210>  59
<211>  20
<212>  DNA
<213>  Artificial Sequence

<220>
<223>  AtACTIN8 ChIP Reverse primer

<400>  59

accggttgta cgaccactgg                                                    20

<210>  60
<211>  253
<212>  PRT
<213>  Arabidopsis thaliana

<400>  60

Met Ser Ser Ser Met Glu Cys Ser Glu Phe Val Gly Ser Arg Arg Phe
1               5                   10                  15

Thr Gly Lys Lys Pro Ser Phe Ser Gln Thr Cys Ser Arg Leu Ser Gln
            20                  25                  30

Tyr Leu Lys Glu Asn Gly Ser Phe Gly Asp Leu Ser Leu Gly Met Ala
        35                  40                  45

Cys Lys Pro Asp Val Asn Gly Thr Leu Gly Asn Ser Arg Gln Pro Thr
    50                  55                  60

Thr Thr Met Ser Leu Phe Pro Cys Glu Ala Ser Asn Met Asp Ser Met
65                  70                  75                  80

Val Gln Asp Val Lys Pro Thr Asn Leu Phe Pro Arg Gln Pro Ser Phe
                85                  90                  95

Ser Ser Ser Ser Ser Ser Leu Pro Lys Glu Asp Val Leu Lys Met Thr
            100                 105                 110

Gln Thr Thr Arg Ser Val Lys Pro Glu Ser Gln Thr Ala Pro Leu Thr
        115                 120                 125

Ile Phe Tyr Ala Gly Gln Val Ile Val Phe Asn Asp Phe Ser Ala Glu
    130                 135                 140

Lys Ala Lys Glu Val Ile Asn Leu Ala Ser Lys Gly Thr Ala Asn Ser
145                 150                 155                 160

Leu Ala Lys Asn Gln Thr Asp Ile Arg Ser Asn Ile Ala Thr Ile Ala
                165                 170                 175

Asn Gln Val Pro His Pro Arg Lys Thr Thr Thr Gln Glu Pro Ile Gln
            180                 185                 190

Ser Ser Pro Thr Pro Leu Thr Glu Leu Pro Ile Ala Arg Arg Ala Ser
        195                 200                 205

Leu His Arg Phe Leu Glu Lys Arg Lys Asp Arg Val Thr Ser Lys Ala

210                 215                 220

Pro Tyr Gln Leu Cys Asp Pro Ala Lys Ala Ser Ser Asn Pro Gln Thr
225                 230                 235                 240

Thr Gly Asn Met Ser Trp Leu Gly Leu Ala Ala Glu Ile
                245                 250

<210>  61
<211>  195
<212>  PRT
<213>  Artificial Sequence

<220>
<223>  JAZ1DeltaJas

<400>  61

Met Ser Ser Ser Met Glu Cys Ser Glu Phe Val Gly Ser Arg Arg Phe
1               5                   10                  15

Thr Gly Lys Lys Pro Ser Phe Ser Gln Thr Cys Ser Arg Leu Ser Gln
            20                  25                  30

Tyr Leu Lys Glu Asn Gly Ser Phe Gly Asp Leu Ser Leu Gly Met Ala
        35                  40                  45

Cys Lys Pro Asp Val Asn Gly Thr Leu Gly Asn Ser Arg Gln Pro Thr
    50                  55                  60

Thr Thr Met Ser Leu Phe Pro Cys Glu Ala Ser Asn Met Asp Ser Met
65                  70                  75                  80

Val Gln Asp Val Lys Pro Thr Asn Leu Phe Pro Arg Gln Pro Ser Phe
                85                  90                  95

Ser Ser Ser Ser Ser Ser Leu Pro Lys Glu Asp Val Leu Lys Met Thr
            100                 105                 110

Gln Thr Thr Arg Ser Val Lys Pro Glu Ser Gln Thr Ala Pro Leu Thr
        115                 120                 125

Ile Phe Tyr Ala Gly Gln Val Ile Val Phe Asn Asp Phe Ser Ala Glu
    130                 135                 140

Lys Ala Lys Glu Val Ile Asn Leu Ala Ser Lys Gly Thr Ala Asn Ser
145                 150                 155                 160

Leu Ala Lys Pro Ile Ser Glu Ala Thr Ser Leu Leu Ser Gln Thr Lys
                165                 170                 175

Phe Leu Ile Gln Glu Lys Pro Gln His Lys Ser Gln Ser Asn Pro Pro
            180                 185                 190

Gln His His
        195

## Description

The invention relates to a nucleotide sequence that improves resistance to biotrophic pathogens, in particular to *Pseudomonas,* preferably to *P. syringae,* without increasing susceptibility to necrotrophs. Therefore, the present invention can be circumscribed to the agricultural field.

### BACKGROUND ART

In plants, preformed physical and biochemical barriers constitute the first line of plant defence against pathogens. Phytopathogenic bacteria must enter by natural surface openings such as stomata or wounds. To defend themselves, plants have evolved sophisticated strategies to perceive their attacker during the infection process and to translate this perception into an effective immune response. Two tiers of recognition by the innate immune system have been defined (Jones, J. D. and Dangl, J. L., Nature 2006; 444(7117): 323-329). One of them is triggered by the recognition of highly conserved microbe-associated molecular patterns (MAMPs) by host cell transmembrane proteins that function as pattern recognition receptors (PRRs), which in turn, activate MAMP-triggered immunity (MTI; (Jones, J. D. and Dangl, J. L. Nature 2006; 444(7117): 323-329)). This activates sufficient defence to resist non-pathogenic microbes and probably also, some pathogens. Plants rapidly close stomata upon perception of MAMPs during pathogen infection to inhibit the entry of pathogen and host tissue colonization (Melotto, M., et al. Cell. 2006; 126(5): 969-980). Weak phytobacterial epiphytics that fail to enter leaf tissues die on the surface of the leave, where conditions are supposed to be difficult for bacterial survival. The bacterial-triggered stomatal closure response, so-called the stomatal defence layer, represents an integral part of plant innate immune system.

*Pseudomonas syringae* (*P. syringae*) is a widespread bacterial pathogen that causes disease on a broad range of economically important plant species. In order to infect, *P. syringae* produces a number of phytotoxins and introduces virulence effector proteins into the plant cell to promote plant susceptibility (Jones, J. D. and Dangl, J. L.. Nature 2006; 444: 323-329; O'Brien, H. E. et al. Annu Rev Phytopathol. 2011; 49: 269-289; Xin, X. F. and He S. Y. Annu Rev Phytopathol. 2013; 51: 473-498). Some *P. syringae* strains have evolved a sophisticated strategy for manipulating the complex hormonal homeostasis in which plant immunity relies on, by producing coronatine (COR), a mimic of the bioactive jasmonic acid (JA) hormone: JA-isoleucine (JA-Ile) (Fonseca, S. et al. Curr Opin Plant Biol. 2009; 12(5): 539-547). In general terms, salicylic acid (SA) signalling mediates resistance against biotrophic and hemi-biotrophic microbes such as *P. syringae,* whereas a combination of JA and ethylene (ET) pathways activates resistance against necrotrophs such as the fungal pathogen *Botrytis cinerea* (Robert-Seilaniantz, A. et al. Annu Rev Phytopathol. 2011; 49: 317-343). SA and JA/ET defence pathways generally antagonize each other and thus, elevated resistance against biotrophs is often correlated with increased susceptibility to necrotrophs, and vice versa (Grant, M. and Lamb, C. Curr Opin Plant Biol. 2006; 9(4):414-20). COR contributes to disease symptomatology by inducing chlorotic (yellow) lesions (Kloek, A. P. et al. Plant J. 2001; 26(5): 509-522, Brooks, D. M. et al. Mol Plant Microbe Interact. 2004; 17(2): 162-174; Uppalapati, S. R. et al. Mol Plant Microbe Interact. 2007; 20(8): 955-965), facilitates entry of the bacteria into the plant host by stimulating the re-opening of stomata after bacterial-triggered stomatal closure (Melotto, M. et al. Cell. 2006; 126(5): 969-980, Melotto, M. et al. Annu Rev Phytopathol. 2008; 46: 101-122), promotes bacterial growth in the apoplast by inhibiting SA-dependent defences required for *P. syringae* resistance, because of its activation of the antagonistic JA pathway (Cui, J. et al. Proc Natl Acad Sci USA. 2005; 102(5): 1791-1796; Laurie-Berry, N. et al. Mol Plant Microbe Interact. 2006; 19(7): 789-800) and enhances bacterial virulence systemically.

JAZ co-receptors are COI1 (COI1 coronatine-insensitive protein 1) substrates that negatively regulate the JA-signalling pathway by directly interacting with and repressing MYC2-like transcription factors (TFs) that control JA-regulated genes (Chini, A. et al. Nature. 2007; 448(7154): 666-671; Thines, B. et al. Nature. 2007; 448(7154): 661-665; Sheard, L. B. et al. Nature. 2010; 468(7322): 400-405; Fernandez-Calvo, P. et al. Plant Cell. 2011; 23(2): 701-715; Pauwels, L. and Goossens, A. Plant Cell. 2011; 23(9): 3089-3100). Repression of TFs by JAZs is mediated by a general co-repressor machinery (Pauwels, L. et al. Nature. 2010; 464(7289): 788-791). The JAZ family of JA-repressors have emerged as central modulators of JA signaling (Chini, A. et al. Nature. 2007; 448(7154): 666-671; Thines, B. et al. Nature. 2007; 448(7154): 661-665; Yan, Y. et al. Plant Cell. 2007; 19(8): 2470-2483). The JAZ family consists of 13 members in Arabidopsis showing different tissue- and stage- specific expression patterns (Chini, A. et al. Nature. 2007; 448(7154): 666-671; Thines, B. et al. Nature. 2007; 448(7154): 661-665; Yan, Y. et al. Plant Cell. 2007; 19(8): 2470-2483).

In spite of the efforts made in order to minimize pathogen infection in plants by manipulating the master defence JA and SA hormonal pathways, current approaches have been unsuccessful due to the well-known antagonism between these pathways. This has been mostly due to the fact that antagonistic interactions between SA and JA pathways would be detrimental to some pathogens but beneficial to others, all of which are present in the field. Consequently, elevated resistance against biotrophs is often correlated with increased susceptibility to necrotrophs, and vice versa (Grant, M. and Lamb, C. Curr Opin Plant Biol. 2006; 9(4):414-20). For instance, dominant mutant versions of JAZ repressors without the Jas domain (JAZdeltaJas) promote JA-insensitivity and, as a consequence, increase resistance to biotrophs but increase susceptibility to necrotrophs (Chini, A. et al. Nature. 2007; 448(7154): 666-671; Thines, B. et al. Nature. 2007; 448(7154): 661-665; Chung, H.S. et al. Phytochemistry. 2009,70(13-14):1547-59). Thus, there is an urgent need to obtain an effective method to protect plants against both biotrophs and necrotrophs, or at least to protect plants against one type of pathogens without increasing the susceptibility to the other type, by overcoming the antagonistic relationship between JA-SA master hormonal defense pathways, that could be realistically transferred to protect crop plants in the field.

### SUMMARY OF THE INVENTION

In the present invention is demonstrated that JAZ2, a family member of the jasmonate zim-domain (JAZ) proteins, is constitutively expressed in stomata guard cells and is required for stomatal closure after bacterial recognition. Additionally, it is also show that in addition to MYC2, MYC3 and MYC4 are also repressed by JAZ2 and regulate the expression of *ANAC19, ANAC55* and *ANAC72* to modulate stomata aperture. Therefore, the results of the present invention demonstrate the existence of a COI1-JAZ2-MYC2,3,4-ANAC19,55,72 module responsible for the regulation of stomatal aperture that is hijacked by bacterial COR to promote infection. More importantly, dominant mutations that make JAZ2 resistant to degradation (hereafter called JAZ2deltaJas or JAZ2ΔJas forms) fully prevent stomatal re-opening by COR and are highly resistant to biotrophs and/or hemi-biotrophs, such as *P. syringae,* and additionally these plants do not show an increase of susceptibility to necrothophs, such as *Botrytis cinerea, Plectosphaerella cucumerina* and/or *Alternaria brassicicola.* Consequently, the inventors solve the trade-off of the antagonics interactions between the SA and JA defense pathway, in which an increase in the resistance to biotrophic pathogens (due to JA-insensitivity/by blocking the JA-signaling pathway) results in an enhanced susceptibility to necrotrophs, and vice versa.

The key issue of this invention is that the JA-dependent signalling is specifically modified at stomata without affecting JA responses at plant leaves (mesophyll cells) and, therefore, without affecting susceptibility to general necrotrophic pathogens, only restricting the entry of biotrophic pathogens that use the stomata as entry ports for invasion. The use in crops of the guard cell specific expression of a modified JAZ nucleic acid, operably linked to a promoter that directs the expression of the nucleic acid to the guard cells of the stomata, wherein said modified JAZ encodes a polypeptide comprising a ZIM domain, but not a functional Jas motif, has the potential to solve the trade-off between resistance to biotrophs and necrotrophs as it uncouples spatially the SA-JA antagonism controlling resistance to both types of pathogens. Solving this trade-off has been a major issue in agriculture.

Remarkably, the above-described modified JAZ2 sequence (hereafter called JAZ2deltaJas) that encodes a JAZ2 variant resistant to degradation confers improved resistance to the biotrophic and/or hemi-biotrophic pathogens, such as *Pseudomonas syringae,* without altering levels of resistance against a broad range of necrotrophic pathogenic fungi such as *Botrytis cinerea, P. cucumerina* and/or *A. brassicicola,* which do not use stomata to enter the host. This discovery also evidence novel cell type specific strategies for crop protection against bacterial biotrophic infections that use stomata as entry ports, without compromising whole leaf resistance to necrotrophic pathogens. This invention is based on the fact that generally, pathogenic biotrophic bacteria enter through stomatal pores while fungal necrotrophic pathogens use direct penetration of the plant leave surface to enter the host by localized secretion of lytic enzymes and/or mechanical force.

In the examples included in the present invention is demonstrated that JAZ2 is a specific JAZ located at the stomata, and that inactivation or deletion of "Jas" domain (domain Jasmonate-specific) of JAZ2 in plants improve resistance to biotrophic and/or hemi-biotrophic pathogens, such as *Pseudomonas syringae* bacteria while also maintaining the resistance to necrotrophs such as *Botrytis cinerea* or any other necrothropic pathogens disclosed in the present invention. As pointed above, dominant mutant versions of JAZ repressors without the functional Jas domain (JAZdeltaJas) promote JA-insensitivity and, as a consequence, increase resistance to biotrophs but also increase susceptibility to necrotrophs (Chini, A. S. et al. Nature. 2007; 448(7154): 666-671; Thines, B. et al. Nature. 2007; 448(7154): 661-665; Chung, H.S. et al. Phytochemistry. 2009,70(13-14):1547-59). The key invention of this work is that combining the inactivation (non-functional) or deletion of the Jas domain described in the present invention together with the specific expression of the modified JAZ sequence at the guard cells of the stomata, results in an increased resistance of the plant to biotrophs, but without the trade-off of enhancing susceptibility to necrotrophs, because the JA-insensitivity promoted by JAZdeltaJas is restricted to guard cells and do not affect mesophyll cells defence.

The JAZ2ΔJas mutant protein does not interact with COl1, and therefore this mutant protein is resistant to proteasome-mediated degradation, so that the resulting plants are insensitive to JA in the stomata. Inventors have conducted comparative experiments with another mutant of the same family, JAZ1 and JAZ3 (eliminating the Jas domain as well) finding that the JAZ2 truncated form or JAZ2 comprising a non-fuctional or inactivated "Jas domain" is surprisingly the one that provides better results for resistance to both types of pathogens due to the specific presence at the guard cells of the stomata of the JAZ2 truncated protein or JAZ2 with a non-functional "Jas domain" but not of the JAZ1 or JAZ3 truncated forms, which are expressed in other tissues.

The JAZ family of JA-repressors are characterized by two highly conserved sequence motifs, namely ZIM (central) and Jas (C-terminal) domains, containing almost all JAZs the functional domains required for function. This indicates that the specific activity of modified JAZ2 protein in restricting bacterial biotrophic invasion compared to any other JAZ is due the specific targeting of JAZ2 to the stomata. Thus, besides the use of JAZ2ΔJas variants that are naturally present at the stomata, it was also establish the use of any other JAZΔJas variant (from all existing JAZ proteins, i.e. JAZ1 to JAZ13) artificially targeted to the stomata through the use of new promoters or modification of natural promoters that would led to expression at guard cells. Experiments with these truncated JAZ proteins have been made in *Arabidopsis thaliana* and it is expected that the same technical effect will be achieved in all plant that have stomata, e.g. tomato, potato, tobacco, canola, rice and in general all higher plants.

Interestingly for the products obtained by the enterprises of the breeding sector, plant mutants can be generated in cultures without transgenesis (e.g. by gene editing techniques such as CRISPR/Cas).

A first aspect of the invention refers to an isolated nucleic acid sequence that comprises a modified *JAZ* nucleic acid, operably linked to a promoter that directs the expression of the nucleic acid to the guard cells of the stomata, wherein said modified *JAZ* encodes a polypeptide comprising a ZIM domain, but not a functional Jas domain; a functional variant of the modified *JAZ* nucleic acid or JAZ polypeptide (*JAZ*/JAZ), a homologue or orthologue thereof. Preferably the modified *JAZ,* or the corresponding modified JAZ polypeptide, is selected from the list consisting of any modified *JAZ1, JAZ2, JAZ3, JAZ4, JAZ5, JAZ6, JAZ7, JAZ8, JAZ9, JAZ10, JAZ11, JAZ12* and *JAZ13* nucleic acids; a functional variant of any modified *JAZ1, JAZ2, JAZ3, JAZ4, JAZ5, JAZ6, JAZ7, JAZ8, JAZ9, JAZ10, JAZ11, JAZ12 and JAZ13* nucleic acid; a homologue of any modified *JAZ1, JAZ2, JAZ3, JAZ4, JAZ5, JAZ6, JAZ7, JAZ8, JAZ9, JAZ10, JAZ11, JAZ12 and JAZ13;* or orthologue of any modified *JAZ1, JAZ2, JAZ3, JAZ4, JAZ5, JAZ6, JAZ7, JAZ8, JAZ9, JAZ10, JAZ11, JAZ12 and JAZ13.*

The acronym JAZ refers to "Jasmonate ZIM-Domain". All *JAZ* proteins contain two highly conserved sequence motifs, the ZIM domain in the central portion of the protein, and the C-terminal Jas motif. In addition, there is a weakly conserved region at the N-terminus. Wide explanations can be found at scientific bibliography to know the function of said domains and motifs.

From now on the isolated nucleic acid of the first aspect of the present invention will be also referred to as the "nucleic acid of the invention". Herein the terms "nucleic acid of the invention", "nucleotide sequence of the invention", "polynucleotide of the invention" and "oligonucleotide of the invention" are used interchangeably.

The modified (non-naturally occurring) *JAZ* nucleic acid of the first aspect of the invention comprises any known *JAZ* nucleic acids, with the proviso that comprise the ZIM domain but not a functional Jas domain.

In a particular embodiment of the present invention the modified *JAZ* nucleic acid or the corresponding modified JAZ polypeptide is from *Arabidopsis thaliana (A. thaliana; At).* Therefore, the modified *JAZ,* or the corresponding modified JAZ polypeptide, is selected from the list consisting of any modified *AtJAZ1, AtJAZ2, AtJAZ3, AtJAZ4, AtJAZ5, AtJAZ6, AtJAZ7, AtJAZ8, AtJAZ9, AtJAZ10, AtJAZ11, AtJAZ12 and AtJAZ13* nucleic acids from *A. thaliana* that comprise the ZIM domain but not a functional Jas domain; a functional variant of any modified *AtJAZ1, AtJAZ2, AtJAZ3, AtJAZ4, AtJAZ5, AtJAZ6, AtJAZ7, AtJAZ2, AtJAZ9, AtJAZ10, AtJAZ11, AtJAZ12 and AtJAZ13* nucleic acid; a homologue of any modified *AtJAZ1, AtJAZ2, AtJAZ3, AtJAZ4, AtJAZ2, AtJAZ6, AtJAZ7, AtJAZ2, AtJAZ9, AtJAZ10, AtJAZ11, AtJAZ12 and AtJAZ13;* or orthologue of any modified *AtJAZ1, AtJAZ2, AtJAZ3, AtJAZ4, AtJAZ2, AtJAZ6, AtJAZ7, AtJAZ2, AtJAZ9, AtJAZ10, AtJAZ11, AtJAZ12 and AtJAZ13.*

In *A. thaliana* the *JAZ* genes codify for the following JAZ proteins:
*JAZ1* gene codifies for JAZ1 protein: Locus At1g19180 (JAZ1 iHOPTIFY10A iHOP, ATJAZ1, JASMONATE-ZIM-DOMAIN PROTEIN 1, TIFY10A).
*JAZ2* gene codifies for JAZ2 protein: Locus At1g74950 (JASMONATE-ZIM-DOMAIN PROTEIN 2, TIFY10B). SEQ ID NO: 1 refers to its cDNA, SEQ ID NO: 2 to the genomic DNA and SEQ ID NO: 3 to the protein.
*JAZ3* gene codifies for JAZ3 protein: Locus At3g17860 (JAI3, JASMONATE-INSENSITIVE 3, JASMONATE-ZIM-DOMAIN PROTEIN 3, TIFY6B).
*JAZ4* gene codifies for JAZ4 protein: Locus At1g48500 (ATJAZ4, JASMONATE-ZIM-DOMAIN PROTEIN 4, TIFY DOMAIN PROTEIN 6A, TIFY6A).
*JAZ5* gene codifies for JAZ5 protein: Locus At1g17380 (JASMONATE-ZIM-DOMAIN PROTEIN 5, TIFY11A).
*JAZ6* gene codifies for JAZ6 protein: At1g72450 (JASMONATE-ZIM-DOMAIN PROTEIN 6, TIFY DOMAIN PROTEIN 11B, TIFY11B).
*JAZ7* gene codifies for JAZ7 protein: Locus At2g34600; Gene ID: 818025; (JASMONATE-ZIM-DOMAIN PROTEIN 7, TIFY5B).
*JAZ8* gene codifies for JAZ8 protein: Locus At1g30135; GenBank: ABG48454.1 (JASMONATE-ZIM-DOMAIN PROTEIN 8, TIFY5A).
*JAZ9* gene codifies for JAZ9 protein: Locus At1g70700 (JASMONATE-ZIM-DOMAIN PROTEIN 9, TIFY7).
*JAZ10* gene codifies for JAZ10 protein: Locus At5g13220 (JAS1, JASMONATE-ASSOCIATED 1, JASMONATE-ZIM-DOMAIN PROTEIN 10, TIFY DOMAIN PROTEIN 9, TIFY9).
*JAZ11* gene codifies for JAZ11 protein: Locus At3g43440 (JASMONATE-ZIM-DOMAIN PROTEIN 11, TIFY3A).
*JAZ12* gene codifies for JAZ12 protein: Locus At5g20900 (JASMONATE-ZIM-DOMAIN PROTEIN 12, TIFY3B).
*JAZ13* gene codifies for JAZ13 protein: Locus At3g22275 (JASMONATE ZIM-DOMAIN PROTEIN 13).

A preferred embodiment of the first aspect of the invention relates to the isolated nucleic acid wherein the ZIM domain comprises the sequence SEQ ID NO: 4 preferably the ZIM domain comprises a sequence with at least 65% identity with SEQ ID NO: 5; a functional variant of the modified *JAZ*/JAZ, a homologue or orthologue thereof.

The ZIM domain is conserved among different plant species and it is a sequence well known by the expert in the field. The SEQ ID NO: 4 relates to a consensus sequence when compared the sequences shown in the table:

| ZIM domain | | |
|---|---|---|
| **SEQ ID** | **Protein** | **Selected ZIM domain sequence** |
| SEQ ID NO: 16 | AtJAZ1 | PLTIFYAGQVIVFNDFSAEKAKEVINLA |
| SEQ ID NO: 5 | AtJAZ2 | PLTIFYGGRVMVFDDFSAEKAKEVIDLA |
| SEQ ID NO: 17 | AtJAZ3 | QLTIFYAGSVCVYDDISPEKAKAIMLLA |
| SEQ ID NO: 18 | AtJAZ4 | QLTIFYAGSVLVYQDIAPEKAQAIMLLA |
| SEQ ID NO: 19 | AtJAZ5 | QLTIFFGGKVLVYNEFPVDKAKEIMEVA |
| SEQ ID NO: 20 | AtJAZ6 | QLTIFFGGKVMVFNEFPEDKAKEIMEVA |
| SEQ ID NO: 21 | AtJAZ7 | ILTIFYNGHMCVSSDLTHLEANAILSLA |
| SEQ ID NO: 22 | AtJAZ8 | RITIFYNGKMCFSSDVTHLQARSITSIA |
| SEQ ID NO: 23 | AtJAZ9 | QLTIFYGGTISVFNDISPDKAQAIMLCA |
| SEQ ID NO: 24 | AtJAZ10 | PMTIFYNGSVSVFQVSRNKAGEIMKVA |
| SEQ ID NO: 25 | AtJAZ11 | QLTIIFGGSFSVFDGIPAEKVQEILHIA |
| SEQ ID NO: 26 | AtJAZ12 | QLTIFFGGSVTVFDGLPSEKVQEILRIA |

The percentage of identity between the ZIM domain of the JAZ2 protein of *A. thaliana* (SEQ ID NO: 5) and other ZIM-domain containing proteins (TIFY-like proteins) from other plants is the following: 71% for *Setaria italica,* 68% for *Malus domestica* and 78% for *Nelumbo nucifera.*

Preferably, the ZIM domain of the nucleic acid of the invention comprises SEQ ID NO: 27 (TIFY sequence wherein F and/or Y amino acids are substituted by conserved amino acids). More preferably, the ZIM domain comprises SEQ ID NO: 28 (TIFY sequence).

The term "identity", as used herein, refers to the percentage of identical nucleic acid residues between two nucleic acid sequences when they are compared. The sequence comparison methods are known in the art, and include but are not limited to BLASTN, and FASTA program ClustalW. The expert in the field can consider that nucleic acid identity percentages of at least 70%, 80%, 85%, 90%, 95%, 96%, 97%, 98% or 99% maintain the same properties and functions of said nucleic acid.

In the present invention when the ZIM domain comprises a sequence with at least 65% identity with SEQ ID NO: 5; it means that the percentage of identity can be 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%.

A more preferred embodiment of the first aspect of the invention relates to the isolated nucleic acid wherein the Jas motif (domain) comprises the sequence SEQ ID NO: 6, preferably comprises a sequence with at least 70% identity with SEQ ID NO: 7; a functional variant of the modified *JAZ* nucleic acid, a homologue or orthologue thereof.

As used herein the term "non-functional", "inactivated" or "inactivation" when referring to a Jas domain (motif) means that the known normal function or activity of this domain has been eliminated or highly diminished. In this sense, wherein these terms are binding to Jas domain means that JAZdeltaJas proteins confer JA-insensitivity in the plants. The residues of the Jas domain involved in the interation with COI1 and JA-Ile have been described in Sheard *et al* (Sheard, L.B. et al. Nature. 2010 (468): 400-405). As used herein, any mutation in one or more of these essential residues should eliminate JAZ binding to COI1 and, thus, render the JAZ mutant resistant to degradation giving rise to a similar phenotype than JAZdeltaJas proteins. Additionally, inactivation which renders the gene or protein, preferably Jas domain, non-functional includes such methods as deletions, mutations, substitutions, interruptions or insertions in the nucleic acid gene sequence.

A "deletion" of a gene as used herein may include deletion of the entire coding sequence, deletion of part of the coding sequence, deletion of the regulatory region, deletion of the translational signals or deletion of the coding sequence including flanking regions.

As used herein the term "mutation" when referring to a nucleic acid refers to any alteration in a nucleic acid such that the product of that nucleic acid is partially or totally inactivated. Examples of mutations include but are not limited to point mutations, frame shift mutations and deletions of part or all of a gene.

As used herein, the term "modified" when referring to nucleic acid or a polynucleotide means that the nucleic acid has been altered in some way as compared to a wild-type, or a naturally-ocurring nucleic acid, such as by mutation in; deletion of part or all of the nucleic acid; or by being operably linked to a transcriptional control region.

The Jas domain is conserved among different plant species and it is a sequence well known by the expert in the field (for example in Thireault, C. et al. The Plant Journal. 2015; 82:669-679). The SEQ ID NO: 6 relates to a consensus sequence when *At*JAZ1-12 were compared according to Thireault, C. *et al.* (Theriault C. et al. The Plant Journal. 2015; 82:669-679).

As it is mentioned previously, JAZdeltaJas proteins disclosed in the present invention, which confer JA-insensitivity have a Jas domain (motif) eliminated, mutated or non-functional. In this sense, Sheard *et al.* (Sheard, L.B. et al. Nature. 2010 (468): 400-405) disclosed that the Jas domain sequence is sufficient for COl1 binding in the presence of the hormone. Consequently, any mutation performed in the at least one essential residues of the Jas domain comprising the SEQ ID NO: 6 or 7 of the present invention, which blocks the interaction with JA-Ile or COl1 are comprised in the present invention. Amino acids esential for the interaction of the Jas motif with COl1 or JA-Ile are described by Sheard L.B. (Sheard, L. B. et al. Nature. 2010; 468(7322): 400-405) for JAZ1.

The percentage of identity between the Jas domain of the AtJAZ2 protein (SEQ ID NO: 7) and other Jas-domain containing proteins from other plants is the following: 86% for *Hevea brasiliensis* and 88% for *Vitis vinifera.*

In the present invention when the Jas domain comprises a sequence with at least 80% identity with SEQ ID NO: 7; it means that the percentage of identity can be 80, 81, 82, 83, 84, 85, 86, 87, 88, 89, 90, 91, 92, 93, 94, 95, 96, 97, 98, 99 or 100%. The nucleic acid sequence described in the first aspect of the present invention can be of any length, preferably between 2 and 1101 nucleotides, more preferably between 2 and 888 nucleotides. In a preferred embodiment the length of nucleic acid is 84 nucleotides.

When the modified JAZ nucleic acid is JAZ2 of *A. thaliana,* a preferred embodiment refers to the isolated nucleic acid that comprises the SEQ ID NO: 1 of any length that comprises at least nucleotides 1-438, preferably 1-603, and has deleted at least nucleotides 604-684. Another preferred embodiment refers to the isolated nucleic acid that comprises the SEQ ID NO: 2 of any length that comprises at least nucleotides 1-849, preferably 1-1101, and has deleted at least nucleotides 1102-1274. In all cases, there will be a minumin N-terminal size that contains the TIFY domain (Or variant thereof as described in SEQ ID NO: 16 to SEQ ID NO: 28) for recruiting NINJA/TOPLESS repressive machinery.

A more preferred embodiment of the first aspect of the present invention relates to the isolated nucleic acid which comprises any of the JAZ nucleic acids disclosed in the present invention selected from the native JAZ1 and JAZ3 to JAZ13, comprising a non-fuctional Jas domain, without their native promoter and with the condition that are operably linked to a promoter that direct the expression of the nucleic acid to the guard cells of the stomata. In a preferred embodiment of the first aspect of the present invention, the promoter that direct the expression of the nucleic acid to the guard cells of the stomata are selected from the group consisting of promoter of *JAZ2* from *A. thaliana* (SEQ ID NO: 11), any regulatory sequence driving gene expression to the stomata such as MYB60 and GC1 among others (Yang, Y., et al. Plant Methods. 2008; 4: 6; Rusconi F., et al. J Exp Bot. 2013; 64(11):3361-71), preferably the promoter is the native promoter of JAZ2 from *A. thaliana* (SEQ ID NO: 11).

A more preferred embodiment of the first aspect of the invention relates to the isolated nucleic acid which comprises a sequence with at least 70% identity with SEQ ID NO: 8, corresponding to the cDNA of the modified *AtJAZ2* (JAZ2ΔJas), or SEQ ID NO: 9, corresponding to the gDNA of the modified *AtJAZ2* (JAZ2ΔJas); a functional variant, a homologue or orthologue thereof. In the present invention the term *JAZ2ΔJas* as synonymous of SEQ ID NO: 8 or 9 is used. The term JAZ2ΔJas is also used as a synonymous of SEQ ID NO: 10.

Herein the terms "nucleotide sequence", "polynucleotide", "nucleic acid" and "oligonucleotide" are used interchangeably.

The nucleotide sequence of the present invention is operably linked to a promoter or regulatory sequence that directs the expression of the nucleic acid to the guard cells of the stomata. Said promoter is selected from any plant promoter directing the expression of the nucleic acid of the invention to the guard cells of the stomata, or from any artificial promoter directing the expression of said nucleic acid to the guard cells of the estomata. A more preferred embodiment of the first aspect of the invention relates to the isolated nucleic acid of the first aspect of the invention wherein the promoter is selected from the list consisting of: promoter of *JAZ1, JAZ2, JAZ3, JAZ4, JAZ5, JAZ6, JAZ7, JAZ8, JAZ9, JAZ10, JAZ11, JAZ12 and JAZ13,* preferably from *A. thaliana.* More preferably the promoter comprising a nucleic acid sequence, wherein said nucleic acid sequence has at least 80% sequence identity to the promoter of *JAZ2* from *A. thaliana* (SEQ ID NO: 11); it means that the percentage of identity can be 80, 81, 82, 83, 84, 85, 86, 87, 88, 89, 90, 91, 92, 93, 94, 95, 96, 97, 98, 99 or 100%. A more preferred embodiment the promoter is the promoter of *JAZ2* from *A. thaliana* (SEQ ID NO: 11). It could be also used any regulatory sequence driving gene expression to the stomata such as MYB60 and GC1 promoters among others (Yang, Y. et al. Plant Methods. 2008; 4: 6; Rusconi F. et al. J Exp Bot. 2013; 64(11):3361-71).

As it is used here, the term "promoter" refers to a region of the DNA upstream from the start point of the transcription of a gene, and particularly, that is able to start the transcription of a gene in a plant cell. Promoters contain specific DNA sequences such as response elements that provide an initial binding site for RNA polymerase and for transcription factors that recruit RNA polymerase. It is recognized by the expert in the field that, a promoter or a regulatory sequence that directs the expression of a nucleic acid has at least all elements essential for transcription of said nucleic acid, including, for example, a TATA box or transcription factor binding sites.

The promoter referred in the present invention is a native or non-native promoter that is functional in plant cells. Examples of promoters include but are not limited to, promoters obtained from plants, virus of plants, or bacteria that can express the nucleic acid of the present invention in plant cells as *Agrobacterium* or *Rhizobium.*

Promoters can be classified, for instance, as inducible and constitutive promoters. The promoter as used in the present invention can be an inducible regulatory element that confers conditional expression in the presence of an inducing agent, increasing the expression compared to the expression of the nucleic acid in the absence of said inducing agent.

The term "operatively linked" refers to a juxtaposition in which the components as well described have a relationship that enables them to work in the intentional way. A polynucleotide operatively linked to any regulatory element that controls the expression of said polynucleotide is linked in such a way that the expression of the coding sequence of the polynucleotide is achieved under conditions compatibles with the regulatory element. The regulatory element contains at least all elements or sequences essential for transcription of said polynucleotide, known by the expert in the field. Said regulatory element may also include a terminator sequence.

The nucleotide sequence of the invention, in addition to the coding sequence, it may have other elements, such as but not limited, introns, noncoding sequences at the 5 'or 3', binding sites for ribosomes, stabilizing sequences, promoters, etc. These polynucleotides may also include further coding sequences for additional nucleic acids, which may be useful, for example, but not limited, to enhance stability of the peptide generated from it or to allow a better purification thereof.

To refer to any of the polynucleotide described in the above aspect and its preferred embodiments of the present invention, the term "polynucleotide of the invention" or "polynucleotide of the present invention" can be used.

The nucleic acid sequence of the present invention may have conservative substitutions, known to the skilled expert. Conservative substitutions lead to a polynucleotide that encodes the same peptide.

The term polynucleotide includes genomic DNA or DNA encoding double or single stranded, ribonucleic acid (RNA), any synthetic and genetically manipulated polynucleotide, and both sense and antisense strands. This includes single-chain molecules and double-stranded, such as DNA-DNA hybrids, DNA-RNA and RNA-RNA.

The nucleotide sequence of the invention can be obtained artificially by conventional cloning methods selection and widely known in the prior art.

The nucleic acid can form part of an expression cassette, comprising the nucleic acid of the invention operably linked to control elements of transcription and/or translation.

A second aspect of the invention relates to a vector comprising the isolated nucleic acid of the first aspect of the invention.

The term "vector", as used herein, refers to a nucleic acid molecule that is capable of transferring nucleic acid sequences contained therein to a cell. Examples of recombinant vectors are linear DNA, plasmid DNA, modified viruses, adenovirus/adeno-associated viruses, and retroviral viral vectors, etc.; all widely described in the literature and which can be used following standard molecular biology techniques or purchased from vendors. Vectors may be introduced by any method known to the skilled artisan, for example, but without limiting, by transfection, transformation or infection of the host cells, such as, but not limited to, plant cells.

A third aspect of the invention relates to a protein encoded by the isolated nucleic acid of the first aspect of the invention. From now on, "the protein of the invention". When the protein of the third aspect of the invention is codified by the modified of *A. thaliana*, a preferred embodiment refers to the protein that comprises the SEQ ID NO: 3 of any length that comprises at least aminoacids 1-146 (containing the TIFY, sequence SEQ ID NO: 28), preferably 1-201, more preferably 1-158; and has deleted or non-functional at least residues 202-228.

A preferred embodiment of the third aspect of the invention refers to the protein as defined in SEQ ID NO: 10 (JAZ2ΔJas).

The invention also provides for variants, analogs, homologues or ortologues of the protein of the invention encoded by the isolated nucleic acid of the first aspect of the invention.

As known by the expert in the field, the present invention encompass not only an specific modified *JAZ* nucleic acid or JAZ polypeptide as described herein, but also functional variants or homologues thereof that do not affect the biological activity and function of the resulting protein.

It is well known in the art that alterations in a nucleic acid sequence which result in the production of a different but conservative amino acid at a given site that do not affect the functional properties of the resulting polypeptide. For example, conservative amino acid changes may be made, which although they alter the primary sequence of the peptide, do not normally alter its function. Conservative amino acid substitutions of this type are known in the art, e. g, changes within the following groups: glycine and alanine; valine, isoleucine and leucine; aspartic acid and glutamic acid; asparagine and glutamine; serine and threonine; lysine and arginine; or phenylalanine and tyrosine.

Modifications (which do not normally affect the primary sequence) include *in vivo* or *in vitro* chemical derivatization of the peptide, e. g., acetylation, carbonation or glycosylation.

Generally, variants or homologues of a particular nucleotide sequence or polypeptide of the invention may be determined by sequence alignment programs known in the art.

"Variants" of the modified *JAZ*/JAZ can differ from naturally occurring nucleic acids/polypeptides by codons encoding conservative amino acid sequence differences or by modifications, which do not affect sequence, or by both.

"Homologues" of modified *JAZ*/JAZ are nucleic acids or polypeptides descenting from a common ancestral nucleic acid sequence. Homologous sequence are either orthologue or paralogue sequence. The term homologue used in the present invention also designates a modified *JAZ*/JAZ orthologue from diferent plant species. Homologous sequences are orthologous if they originated by vertical descent from a single gene of the last common ancestor, that is, the copies of a single gene/protein in the two resulting species with a common ancestor sequence are orthologous sequences. Homologous sequences are paralogous if they were originated by a duplication event within the genome of particular specie. That is, the duplication of a gene in an organism occupying two different positions in the same genome.

For example, homologues of *JAZ* could be modified in order to obtain the modified nucleic acid or the modified polypeptide of the present invention from any of the following, not limitating, list of nucleic acids:
*Theobroma cacao* JAZ3 (TCM_012913, TCM_011017), *Theobroma cacao* JAZ6 (TCM_029176), *Theobroma cacao* JAZ12 (TCM_006179, TCM_037429), *Medicago truncatula* JAZ (MTR_5g013530, MTR_2g042900, MTR_6g069870, MTR_4g124960, MTR_8g021380, MTR_1g031930, MTR_8g107300, MTR_5g013515, MTR_2g019190, MTR_5g013520, MTR_4g124950), *Theobroma* cacao JAZ10 (TCM_008419), *Theobroma cacao* JAZ8 (TCM_029461), *Theobroma* cacao JAZ1 (TCM_037495), *Solanum lycopersicum* JAZ3 (LOC100191114), *Solanum lycopersicum* JAZ1 (LOC100134911).

Homologues of JAZ protein could be modified in order to obtain the modified polypeptide of the present invention from the nucleic acid encoding any of the following, not limitating proteins:
*Catharanthus roseus* JAZ (ACM89458, ACM89457), *Salvia miltiorrhiza* JAZ3 (AHK23660), *Salvia miltiorrhiza* JAZ2 (AHK23659), *Nicotiana attenuata* JAZ (AFL46177, AFL46176, AFL46175, AFL46174, AFL46173, AFL46172, AFL46171, AFL46170, AFL46169, AFL46168, AFL46167, AFL46166, AFL46165, AFL46178), *Phaseolus lunatus* JAZ (AIT38287, AIT38286, AIT38285, AIT38283, AIT38282, AIT38281, AIT38275), *Sonneratia apetala* JAZ (AFU90901), *Sonneratia lanceolata* JAZ (AFU90900), *Sonneratia ovata* JAZ (AFU90899), *Sonneratia caseolaris* JAZ (AFU90898), *Sonneratia alba* JAZ (AFU90897), *Phaseolus lunatus* JAZ (AIT38274, AIT38273.1, AIT38272), *Solanum lycopersicum* JAZ3 (NP_001234373, XP_010317740), *Solanum lycopersicum* JAZ1 (NP_001234883, ABU88421), *Catharanthus roseus* JAZ10 (ALI87033), *Catharanthus roseus* JAZ8 (ALI87032), *Catharanthus roseus* JAZ3 (AL187031), *Oryza sativa* JAZ (BAT08274), *Gossypium barbadense* JAZ10 (AJT58397), *Hevea brasiliensis* JAZ (ADI39634), *Gossypium barbadense* JAZ2 (AJT58398), *Hevea brasiliensis* JAZ2 (AIY25007), *Vitis rupestris* JAZ2 (AEP60133), *Vitis quinquangularis* JAZ2 (AFJ23868.1), *Artemisia annua* JAZ2 (AJK93413), *Pyrus pyrifolia* JAZ1 (AGZ89627), *Prunus pérsica* JAZ1 (ADU76348), *Nicotiana tabacum* JAZ1 (ADZ48593), *Genlisea aurea* JAZ1 (EPS61226), *Artemisa annua* JAZ1 (AJK93412), *Hevea brasiliensis* JAZ11 (AIY25012), *Eutrema halophilum* JAZ12 (BAJ34102), *Gossypium barbadense* JAZ13 (AJT58403), *Brassica napus* JAZ (XP_013682524, XP_013682522, XP_013648884, XP_013648883), *Brassica oleracea* JAZ (XP_013590008, XP_013590007, XP_013618140), *Gossypium raimondii* JAZ (XP_012477278, XP_012477277), *Gossypium arboreum* JAZ (KHG27959), *Brassica rapa* JAZ (XP_009128069), *Eucalyptus grandis* (KCW79370, KCW79369, XP_010047457, *Elaeis guineensis* (XP_010939885, XP_010937308), *Nelumbo nucifera* (XP_010273865), *Populus euphratica* (XP_011039955), *Glycine max* TYFY 10A-like (XP_006587054, XP_003546514, NP_001276248), *Glycine soja* TIFY 10A-like (KHN12298, KHN07885), *Vitis vinifera* (CB127776), *Vitis vinifera* TIFY 10A-like (XP_002277157, XP_002272363), *Cicer arietinum* TIFY 10A-like (XP_004488102), *Lotus japonica* (AFK35276), *Ricinus communis* (XP_002516243), Zea *mays* (NP_001182812, XP_002462448, NP_001130163), *Malus domestica* TIFY 10A-like (XP_008337828, XP_008388962), *Phoenix dactylifera* TIFY 10A-like (XP_008784340), *Citrus clementina* (XP_006452845), *Jatropha curcas* TIFY 10A-like (XP_012077082.1). In the list are included other sequences of the JAZ family from maize, rice, wheat, oilseed rape/canola, sorghum, soybean, sunflower, alfalfa, potato, tomato, tobacco, grape, barley, pea, bean, field bean, lettuce, cotton, sugar cane, sugar beet, broccoli or other vegetable brassicas or poplar among many others. In the list are also included other sequences of the JAZ family from ornamental plants, plants useful in timber (wood) production (such as pine tree, oak, chestnut or beech tree) and fruit trees (for example orange tree, pear tree, apple tree, olive tree, cherry tree or peach tree).

The nucleotide sequences of the present invention can be isolated from any plant, particulartly from any crop plant, but can be also from ornamental plants, timber plants and fruit plants. The sequences can be identified by means techniques known in the art (e.g. PCR) and isolated the entire sequence or fragments thereof.

A "fragment" of a polypeptide is included within the present invention if it retains substantially the same activity as the protein of the third aspect of the invention.

Herein the terms "amino acid sequence", "polypeptide", "peptide" and "oligopeptide" are used interchangeably.

A fourth aspect of the invention relates to a host cell comprising the isolated nucleic acid of the first aspect of the invention, or the vector of the second aspect of the invention, or the protein of the third aspect of the invention, with the proviso that when the host cell comprises the protein, the host cell is a plant cell but not an *Arabidopsis thaliana* cell, and specifically, the host cell is a guard cell of the stomata.

The cell can be named as "host cell". The host cells as it is used in the present invention relates to any procariotic or eukaryotic cell or viruses that replicate in prokaryotic or eukaryotic cells. Essentially, it refers to a eukaryotic plant cell and within this group, more preferably, those cells belonging to the Kingdom Plantae, wherein any of these cells comprises the polynucleotide of the invention, or the vector of the invention. Thus, the term plant cell comprises at least one cell of the parenchyma, meristematic cell or of any kind of plant cell, differentiated or undifferentiated. Preferably the plant cell is the guard cell of the stomata, or the mesophyll cells.

To refer to any host cell comprising the polynucleotide of this invention, the term "host cell of the invention" or "host cell of the present invention" can be used.

Viral hosts for expression of the proteins of the present invention include viral particles, which replicate in prokaryotic host or viral particles, which infect and replicate in eukaryotic hosts. Procedures for generating a vector for delivering the isolated nucleic acid or a fragment thereof are well known. Suitable vectors include, but are not limited to, disarmed *Agrobacterium* tumor inducing (Ti) plasmids (e. g., pBIN19) containing a target gene under the control of a vector, such as the cauliflower mosaic (CaMV) 35S promoter or its endogenous promoter, tobacco mosaic virus and the like.

Once the vector has been prepared for expression, the nucleic acid may be introduced or transformed into an appropriate host. Various techniques may be employed for the transformation such as protoplast fusion, calcium phosphate precipitation, electroporation, or other conventional techniques. As is well known, viral sequences may be directly transformed into a susceptible host or first packaged into a viral particle and then introduced into a susceptible host by infection. After the cells have been transformed with the vector, or the virus or its genetic sequence is introduced into a susceptible host, the cells are grown in media and screened for appropriate activities. Expression of the sequence results in the production of the protein of the third aspect of the invention.

Numerous procedures are known in the art to assess whether a transgenic plant comprises the isolated nucleic acid of the first aspect of the invention or the vector of the second aspect of the invention, and need not be reiterated.

Cells which have stably integrated the nucleic acid of the presen invention into their chromosomes can be selected by also introducing one or more reporter genes or markers which allow for selection of host cells which contain the expression vector. The reporter gene or marker may complement an auxotrophy in the host (such as leu2, or ura3, which are common yeast auxotrophic markers), biocide resistance, e. g., antibiotics, or resistance to heavy metals, such as copper, or the like. The selectable marker gene can either be directly linked to the DNA gene sequences to be expressed, or introduced into the same cell by co-transfection.

The present invention further relates to a cell culture comprising the host cell of the invention. To refer to any cell culture comprising the host cell of the present invention, the term "cell culture of the invention" or "cell culture of the present invention" can be used.

The term "cell culture" refers to a cultivation of cells isolated from the same or different type of tissue, or a collection of these cells organized in parts of a plant or in tissues (tissue culture). Types of this kind of cultures are, e.g. but without any limitation, a culture of protoplasts, calli (groups of plant cells undifferentiated able to regenerate a complete plant with the appropriate organogenic program) or a culture of plant cells that are isolated from plants or parts of plants such as embryos, meristematic cells, pollen, leaves or anthers.

A fifth aspect of the invention relates to a plant comprising the isolated nucleic acid of the first aspect of the invention, or the vector of the second aspect of the invention, or the protein of the third aspect of the invention; with the proviso that when the host cell of the plant comprises the protein the host cell is a guard cell of the stomata and wherein the plant is not *Arabidopsis thaliana.*

The plant of the fifth aspect of the present invention can be generated by transgenesis, genome editing technologies, truncation of its own Jas motif in the ZIM-domain containg proteins, chemical random mutagenesis and selection of the mutant, or by selection of T-DNA mutants. As new genome editing technologies, technologies such as ZFNs, TALENs or CRISPR/Cas (Boch, J. et al. Science. 2009; 326(5959): 1509-1512; Christian, M. et al. Genetics. 2010; 186(2): 757-761; Belhaj, K. et al. Plant Methods. 2013; 9(1): 39) could be used to artificially generate the nucleic acid of the present invention in crops such as maize, rice, wheat, oilseed rape/canola, sorghum, soybean, sunflower, alfalfa, potato, tomato, tobacco, grape, barley, pea, bean, field bean, lettuce, cotton, sugar cane, sugar beet, broccoli or other vegetable brassicas or poplar among many others for example, ornamental plants, plants useful in timber (wood) production (such as pine tree, oak, chestnut or beech tree) and fruit trees (for example orange tree, pear tree, apple tree, olive tree, cherry tree or peach tree). Preferably, the plant of the invention is a tomato plant.

By "transgenic plant" as used herein, is meant a plant, plant cell, tissue, flower, organ, including seeds, progeny and the like, or any part of a plant, which comprises an exogenously inserted gene or a targeted genomic modification to one or more alleles of an endogenous gene where the product of the gene produces a product that results in plant improved resistance to biotrophic pathogens without increasing susceptibility to necrotrophs. The manipulated gene herein is designated as "transgene", independently of whether the gene has been introduced exogenously or the endogenous gene has been manipulated; in both cases, the sequence defined as "transgene" has not been shown to be naturally occurring.

The non-transgenic, wild-type, non-transgenic plants or wild type plants, as used herein, mean a plant without said genetic modification. These terms can refer to a cell or a part of a plant such as an organelle like a chloroplast or a tissue, in particular a plant, which lacks said genetic modification but is otherwise as identical as possible to the plants with at least one genetic modification employed in the present invention. The transgenic transcription product may also be oriented in an antisense direction.

The generation of transgenic plants comprising the isolated nucleic acid of the first aspect of the invention (in *sense* or *antisense* orientation) may be accomplished by transforming the plant with a plasmid, liposome, or other vector encoding the desired DNA sequence. Such vectors would, as described above, include, but are not limited to the disarmed *Agrobacterium* tumor-inducing (Ti) plasmids containing a sense or antisense strand. Methods of generating such constructs, plant transformation and plant regeneration methods are well known in the art once the sequence of the gene of interest is known.

The plant of the invention can be achieved by genetic transformation mediated by biolistic, *Agrobacterium tumefaciens* or any other technique known by the skilled in the art (e.g. transformation of protoplasts), that will allow the integration of the polynucleotide of the invention in any of the DNA of the plant; genomic, chloroplastic or the polynucleotide of the invention by crossing and selection.

The use of the CRISPR (Clustered Regularly Interspaced Short Palindromic Repeats)/Cas system to modify/edit the endogenous JAZ genes, preferably the endogenous JAZ2 gene, in any plant species requires the transformation of the plant with a suitable vector that expresses the CRISPR/Cas nuclease and the guiding RNA (gRNA) complementary to a part of the JAZ sequence, preferably JAZ2 sequence, as known by the expert in the field. Examples of this technique can be found in US2014273235A1.

The production of a plant can be performed by means of techniques known by the skilled in the art, for instance:
- The cultivation of embryos: isolation of zygotic embryos promoting their growth as plant in an artificial environment
- Somatic embryogenesis: production of embryos from somatic tissues, such as microspores or leaves
- Organogenesis: production of organs such as stems or roots from various tissues of the plant.

The plant of the present invention can be obtained according with other microbiologic processes as for example:
- Obtaining cybrids: it is produced a cell with its cytoplasm and the cytoplasm of the other cell and this cell can be grown in a suitable medium to produce a plant which expresses the heterologous polynucleotide.
- Fusion of somatic cells (preferably protoplast fusion): at the cytoplasmic level hybrid plants can be the result of: (a) the sum of the cytoplasm of both parental; (b) the cytoplasm of a single parental; (c) a cytoplasm hybrid result of recombination of the genomes extranuclears of both cells. The fusion of somatic cells can be applied; to overcome the incompatibility in interspecific crosses, or a better utilization of the interspecies variation and extraspecific in interspecific compatible crosses.

The plant in the present invention can be any plant with stomata. Preferably the plant is a plant that can be infected by any biotroph pathogen through the stomata. Preferably the plant is any tree propagated or cultured by cuttings, such as fruit trees (for example orange tree, pear tree, apple tree, olive tree, cherry tree, apricot or peach tree); crops such as maize, rice, wheat, oilseed rape/canola, sorghum, soybean, sunflower, alfalfa, potato, tomato, tobacco, grape, barley, pea, bean, field bean, lettuce, cotton, sugar cane, sugar beet, broccoli or other vegetable brassicas or poplar beans cassava, coton, cucurbits, pepper and cereals; ornamental plants; or plants useful in timber (wood) production (such as pine tree, oak, chestnut or beech tree), wherein the plant has resistance to biotrophs, preferably to biotrophic bacteria such as *Pseudomonas syringae, Ralstona, Enwinia* and *Xanthomonas* species, and necrotrophs, preferably *Botrytis cinerea, Fusarium, Plectosphaerella cucumerina* and *Alternaria brassicicola,* among many others.

In accordance with the present invention, plants included within its scope include both higher and lower plants of the Plant Kingdom. Mature plants, including rosette stage plants, and seedlings are included in the scope of the invention. A mature plant, therefore, includes a plant at any stage in development beyond the seedling. A seedling is a very young, immature plant in the early stages of development.

Preferred plants of the present invention, but are not limited to, high yield crop species (including monocots and dicots, e. g., alfalfa, cashew, cotton, peanut, fava bean, french bean, mung bean, pea, walnut, maize, potato, sugar beet, tobacco, oats, wheat, barley and the like), or engineered endemic species. Particularly preferred plants are those from: the Family *Umbelliferae,* particularly of the genera *Daucus* (particularly the species *carota,* carrot) and *Apium* (particularly the species *graveolens,* celery) and the like; the Family *Solanacea,* particularly of the genus *Solanum,* particularly the species *lycopersicum* (tomato), *tuberosum* (potato) and *melongena* (eggplant or aubergine), and the like, and the genus *Capsicum,* particularly the species *annuum* (pepper) and the like; and the Family *Leguminosae,* particularly the genus *Glycine,* particularly the species max (soybean) and the like; and the Family *Cruciferae,* particularly of the genus *Brassica,* particularly the species *rapa* (turnip) and *oleracea* (red cabbage, cauliflower or broccoli) and the like; the Family *Compositae,* particularly the genus *Lactuca,* and the species *sativa* (lettuce), and the like.

By "plant" as used herein, is meant any plant and any part of such plant, be it wild-type, treated, genetically manipulated or recombinant, including transgenic plants. The term broadly refers to any and all parts of the plant, including plant cell, tissue, flower, leaf, stem, root, organ, and the like, and also including seeds, progeny and the like, whether such part is specifically named or not.

A preferred embodiment of this invention refers to any plant described above wherein the expression of the polynucleotide of the invention is greater than the expression of the homologous native gene *JAZ2* under the control of promoters targeting the *JAZ* nucleic acid of the present invention to the stomata, for example using the promoter MYB60.

The plant contains the polynucleotide of the invention in homozygosis, heterozygosis or hemizygosis. As used herein the terms "Homozygous" or "Homozygosity" are understood within the scope of the invention and refer to a plant which is homozygous for a particular gene when identical alleles (forms of a given gene) of the gene are present on both homologs. As used herein, the term "homozygote" refers to an individual cell, plant or germplasm having the same alleles at one or more loci in homologous chromosomal segments. As used herein the terms "Heterozygous" or "Heterozygosity" are understood within the scope of the invention and refer to a plant which is heterozygous for a particular gene when two different alleles occupy the gene's position on the homologous chromosomes. As used herein, the term "heterozygote" refers to a diploid or polyploid individual cell, plant or germplasm having different alleles present at least at one locus. As used herein, the term "hemizygous" or "hemizygosis" refers to a genetic condition of a cell, tissue or organism where a gene or a nucleotic sequence is present in one of the homologous chromosomes, but is not found at the corresponding locus of the other homologous chromosome. For example, a plant which is said to be hemizygous at a given locus for a gene of interest or for a certain sequence only contains the said transgene or the said sequence at that locus of one but not the other one of the respective pair of homologous chromosomes.

A sixth aspect of the present invention refers to a germplasm of the plant of the fifth aspect of the invention. Additionally, the germplasm of the present invention comprising the isolated nucleic acid, the protein, or the host cell of the invention with the proviso that the germplasm is not an *A. thaliana* germplasm.

The germplasm is defined by the biological material that contains the intraspecific genetic variability or by the genetic materials that can perpetuate a species or a population of said plant. Particularly, germplasm refers to a genetic material of or from an individual (e.g., a plant), a group of individuals (e.g., a plant line, variety or family), or a clone derived from a line, variety, species, or culture. The germplasm can be part of an organism or cell, or can be separate from the organism or cell. In general, germplasm provides genetic material with a specific molecular makeup that provides a physical foundation for some or all of the hereditary qualities of an organism or cell culture. Thus germplasm includes cells, seed, tissue culture for any part of the plant from which new plants may be grown, or plant parts, such as leafs, stems, pollen, seed, or cells that can be cultured into a whole plant, or plants established in *ex situ* collections, without excluding any other material in the scope of this definition.

The pollen has high level of interest since the transmission of the genetic and phenotypic characters can be carried out by the pollination of any plant variety compatible with the pollen that is referenced. In this way can be produce a plant which includes the polynucleotide of the invention, after the respective cross and selection, it can be obtained a plant in which the polynucleotide integrates a suitable number of copies in stable condition in order to obtain the same desirable phenotype in the subsequent generations.

A preferred embodiment of the present invention refers to a plant comprising the isolated nucleic acid of the first aspect of the invention, wherein said plant is obtainable by introgression from the plant of the fifth aspect of the invention, or from the germplasm of the sixth aspect of the invention, and preferably the plants are inbred or hybrid plants.

The present invention also refers to a plant obtainable by means the introgression of the modified *JAZ* nucleic acid from the plant of the fifth aspect of the invention, or from the germplasm of the sixth aspect of the invention in any plant having a promoter that directs the expression of the nucleic acid to the guard cells of the stomata, with the proviso that the modified *JAZ* nucleic acid of the obtained plant was operably linked to said promoter that directs the expression of the nucleic acid to the guard cells of the stomata, and preferably the plants are inbred or hybrid plants.

A seventh aspect of the present invention refers to a use of the isolated nucleic acid of the first aspect of the invention, or the vector of the second aspect of the invention, or the protein of the third aspect of the invention, or the cell of the fourth aspect of the invention to produce a plant with resistance to biotrophic or hemi-biotrophic plant pathogens, preferably *Pseudomonas syringae* (for example pathovars *tomato* or *maculicola*), and without modifying the level of susceptibility of said plant to necrotrophic plant pathogens, preferably *Botrytis cynerea.* The term "hemi-biotrophic" is well known in the art. The term "without modifying the level of susceptibility" as used in the present invention means that the tolerance and/or resistance is not substancially altered.

In the present invention among biotrophic or hemi-biotrophic plant pathogens the following can be found: examples of biotrophic or hemi-biotrophic pathogens include *Pseudomonas sp (for example Pseudomonas solanacearum and Pseudomonas syringae,* specifically pathovars *tomato* and *maculicola), Xanthomonas sp (Xanthomonas campestris,* specifically pathovars *campestris* and *vesicatoria), Hyaloperonospora sp, Erisyphe sp, Ralstonia sp, Enwinia sp* and *Magnaporthe sp.* Many other such examples are well known in the art.

In the present invention among necrotrophic plant pathogens the following can be found: *Botrytis cinerea, Fusarium sp., Alternaria brassicicola, Plectosphaerella cucumerina, Phytophthora infestans, Peronospora parasitica, Rhizoctonia solani, Phoma lingam* (*Leptosphaeria maculans*), and *Albugo candida.* Many other such examples are well known in the art.

An eigth aspect of the present invention refers to a method for producing a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea,* comprising:
a. transferring to the isolated plant material the isolated nucleic acid sequence of the frist aspect of the invention, wherein said transfer of said nucleic acid is performed by transformation, by protoplast fusion, by a doubled haploid technique, by gene gun, by electroporation, by viral transduction, or by embryo rescue, provided that when said method involves a doubled haploid technique said method is not essentially biological;
b. identifying the plant material obtained in the step (a) comprising the modified JAZ nucleic acid operably linked to a promoter that directs the expression of the nucleic acid to the guard cells of the stomata, as defined in the first aspect of the invention;
c. growing the plant material identified in the step (b) in a suitable medium to produce at least a plant and/or a germplasm which expresses the modified *JAZ* nucleic acid sequence.

A ninth aspect of the present invention refers to a method for producing a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea,* comprising:
a. modifying the *JAZ* native nucleic acid of the isolated plant material in order to obtain the nucleic acid encoding the Jas domain as defined in the nucleic acid of the first aspect of the invention by means of the Zinc finger nuclease 1 and 2 (ZFN1 and 2) technology, TALENS or a CRISP/Cas technology;
b. identifying the plant material obtained in the step (a) having the nucleic acid encoding a non-functional Jas domain,
c. growing the plant material identified in the step (b) in a suitable medium to produce at least a plant and/or a germplasm which expresses the modified *JAZ* nucleic acid sequence.

A tenth aspect of the present invention refers to a plant obtained by a method of the eigth or the ninth aspect but not *A. thaliana* plant. Preferably, the plant of the invention is a tomato plant.

An eleventh aspect of the present invention refers to a method for detecting a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea,* that comprises detecting the modified *JAZ* nucleic acid operably linked to a promoter that directs the expression of the nucleic acid to the guard cells of the stomata, as defined in the first aspect of the invention.

The detection of the modified *JAZ* nucleic acid operably linked to a promoter that directs the expression of the nucleic acid to the guard cells of the stomata can be carried out by means techniques known in the art (e.g. PCR).

The present invention also refers to a kit that comprises the nucleic acid sequence of the first, the vector of the second aspect of the present invention, or the protein of the third aspect of the present invention or the cell of the fourth aspect of the present invention artificially introduced by using the new genome editing technologies, such as ZFNs, TALENs or CRISPR/Cas in selected crops with appropiate vectors. The kit can also comprise sequences to express the CRISPR/Cas nuclease and/or the sequence for the guiding RNA that is complementary to a part of the modified JAZ sequence. Also the kit can comprise the means to use the ZFNs and/or TALENs technology.

The present invention also refers to the use of the kit of the invention for the production of a plant with resistance to biotrophs, preferably to biotrophic or hemi-biotrophic plant pathogens, preferably to *Pseudomonas syringae, Ralstonia, Enwinia* and *Xanthomonas* species, and without modifying the level of susceptibility of said plant to necrotrophic plant pathogens, preferably *Botrytis cinerea, Fusarium sp.* and *Alternaria brassicicola,* among many others.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

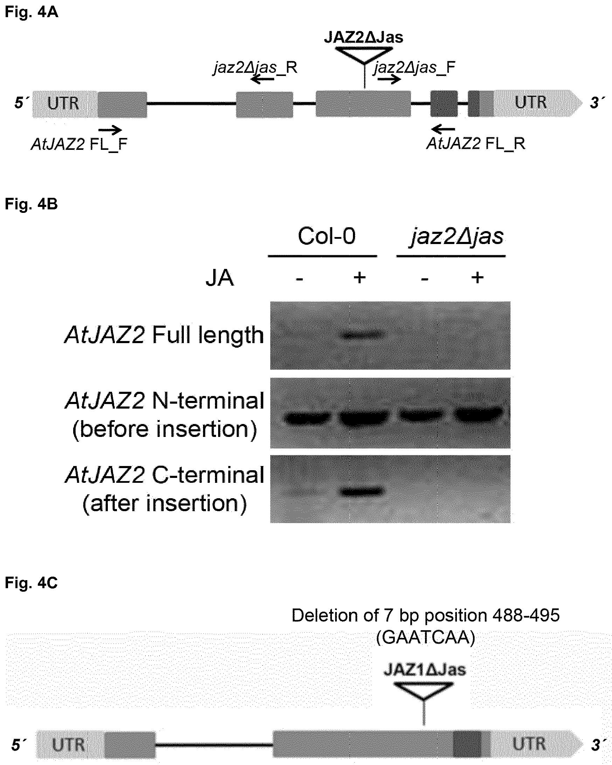

**Figure 1****. Tissue Expression Patterns of JAZ2.**
   Histochemical GUS activity of 6 days old Arabidopsis transgenic seedlings expressing the GUS reporter gene under the control of the JAZ2 promoter (pJAZ2:GUS). Seedlings were treated with 50 µM of JA or mock for 2 hours. GUS activity was detected after overnight staining.
**Figure 2****. Isolation of the *Arabidopsis JAZ2-3* mutant.**
   **(A)** *JAZ2* gene model showing *JAZ2-3* transposon insertion sites. **(B)** RT-PCR to detect full-length *JAZ2* transcript accumulation in WT and *JAZ2-3* mutant after treatment with 1 µM COR (+) or a mock solution (-). **(C)** Quantitative RT-PCR to detect *JAZ2* transcript accumulation in WT and *JAZ2-3* plants after treatment with 1 µM COR or a mock solution using primers that amplify an *AtJAZ2* C-terminal fragment located after the insertion.

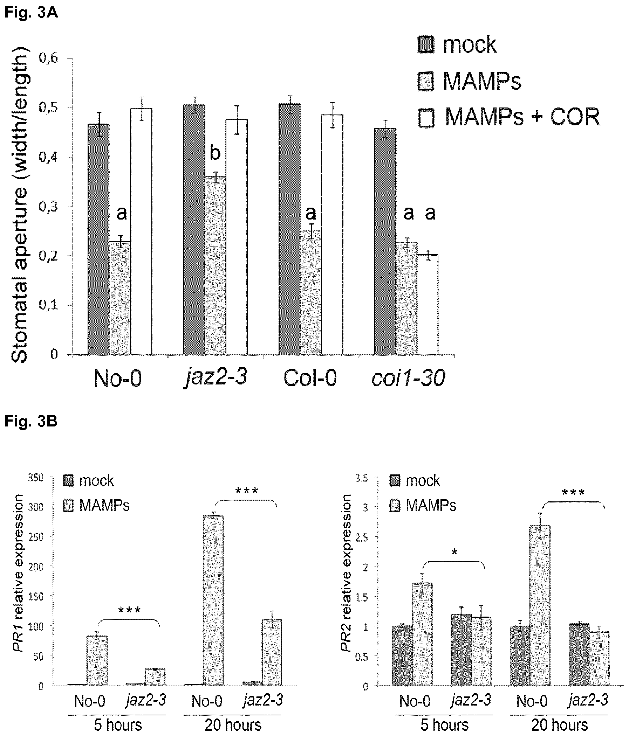

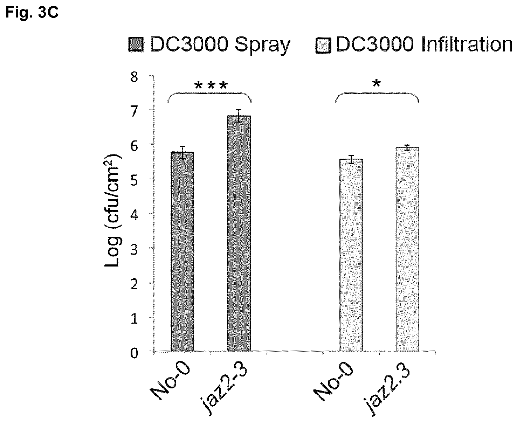

**Figure 3****. JAZ2 regulates COR-induced stomatal dynamics during *Pseudomonas* infection**
   **(A)** Stomatal aperture in *Arabidopsis* No-0, No-0 *JAZ2-3,* Col-0 and Col-0 *coi1-30* leaves measured after 5 hours of incubation with OD₆₀₀=2 of MAMPs, OD₆₀₀=2 of MAMPs and 2 µM of COR or a mock control. Error bars indicate standard error of the mean (SEM) (*n* = 20). Different letters above columns indicate significant differences compared with mock-treated No-0 control sample (Student's *t* test, *p*<0.001). The results are representative of four independent experiments. **(B)** Quantitative RT-PCR analysis of *PR1* and *PR2* expression on Arabidopsis No-0 and *JAZ2.3* seedlings induced for 5 or 20 hours with MAMPs (OD₆₀₀=1.5 of boiled *Pto* DC3000) or a mock control. The measurements (three technical replicates) represent the ratio of expression levels between each sample and mock treated No-0 at each time point. All samples were normalized against the housekeeping gene *AtACT8.* Error bars represent standard deviation (SD). The results are representative of two independent experiments. Asterisks indicate statistically significant differences in the gene induction between MAMP induced Arabidopsis No-0 and *JAZ2.3* lines as indicated (Student's t test, *p<0.05; **p<0.01; ***p<0.001). **(C)** Growth of *Pto* DC3000 on Arabidopsis No-0 and No-0 *JAZ2-3* plants three days after spray inoculation with bacteria at 10⁸ colony-forming units mL⁻¹ (cfu/ml) or syringe infiltration with bacteria at 5x10⁵ cfu/ml. Error bars indicate SEM (n = 7). Asterisks indicate statistically significant values in *JAZ2-3* lines compared to its No-0 WT control (Student's t test, *p<0.05; **p<0.01; ***p<0.001). The results are representative of three independent experiments
**Figure 4****. Isolation of the *JAZ2ΔJas* and *JAZ1ΔJas* mutants.**
   **(A)** *JAZ2* gene model showing the T-DNA insertion site in *JAZ2ΔJas.* The Jas motif coding region is shown in dark grey. **(B)** RT-PCRs to detect *JAZ2* transcript accumulation in WT (Col-0) and *JAZ2ΔJas* Arabidopsis mutants after treatment with 50 µM JA (+) or a mock solution (-) for 2 hours. Three different regions of JAZ2 were amplified to characterize the *JAZ2ΔJas* Arabidopsis mutant: an AtJAZ2 full-length fragment, an AtJAZ2 N-terminal fragment located before the insertion and finally, an AtJAZ2 C-terminal fragment located after the insertion. **(C)** *JAZ1* gene model showing the T-DNA insertion in *JAZ1ΔJas.* The Jas motif coding region is shown in dark grey.

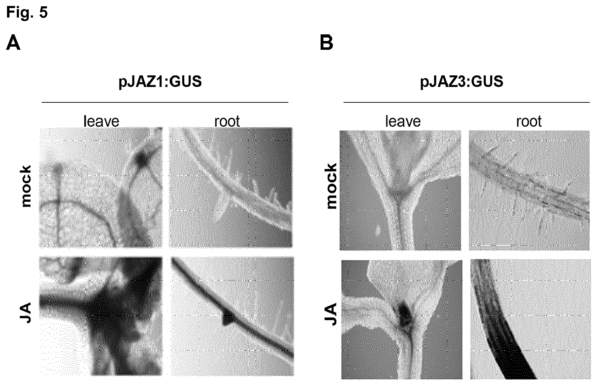

**Figure 5****. Tissue Expression Patterns of *JAZ1* and *JAZ3.***
   Histochemical GUS activity of 6 days old Arabidopsis transgenic seedlings expressing the GUS reporter gene under the control of the promoter of *JAZ1* (*pJAZ1*:GUS) **(A)** or *JAZ3* (*pJAZ3*:*GUS*) **(B)**. For treatment with the hormone (JA panels), the seedlings were treated with 50 µM of JA for 2 hours. GUS activity was detected after overnight staining.

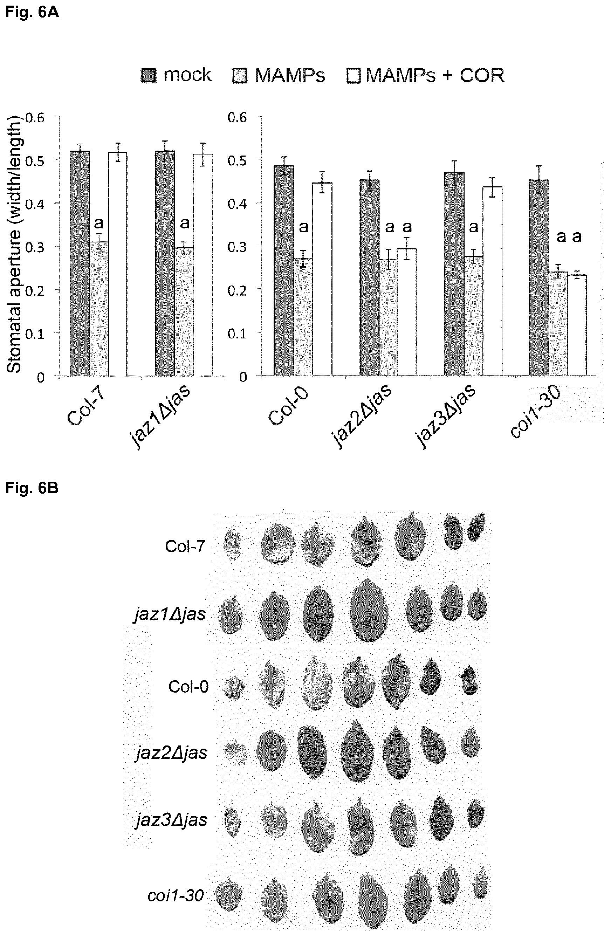

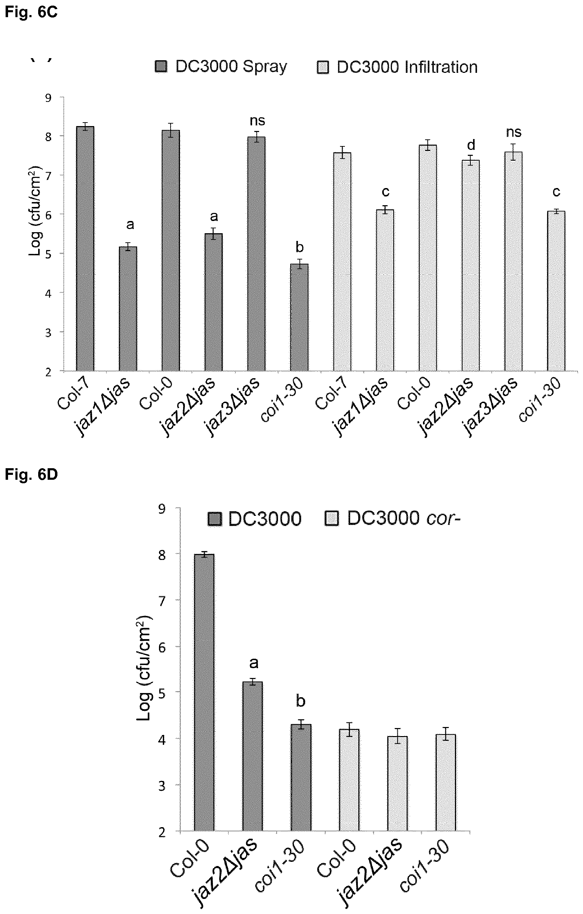

**Figure 6****. Dominant *JAZ2ΔJas* mutants are impaired in COR-induced stomata reopening and resistance to P. syringae infections.**
   **(A)** Stomatal aperture in Arabidopsis Col-7, *JAZ1ΔJas,* Col-0, *JAZ2ΔJas, JAZ3ΔJas* and *coi1-30* leaves measured after 5 hours of incubation with MAMPs (OD₆₀₀=1.5 of crude *Pto* DC3000 extracts), MAMPs plus 2 µM COR or a mock control. Error bars indicate SEM (n = 20). Different letters above columns indicate significant differences compared to their respective mock-treated WT control sample (Col-7 or Col-0) (Student's t test, p<0.001). The results are representative of three independent experiments.
   **(B)** *Pto* DC3000 disease symptoms on Col-7, *JAZ1ΔJas,* Col-0, *JAZ2ΔJas, JAZ3ΔJas* and *coi1-30* plants after spray inoculation with *Pto* DC3000 bacteria at 10⁸ cfu/ml. Pictures were taken 4 days after inoculation and show typical chlorosis caused by *Pto* DC3000. Leaves show representative symptoms of three independent experiments.
   **(C)** Growth of *Pto* DC3000 on Arabidopsis Col-7, *JAZ1ΔJas,* Col-0, *JAZ2ΔJas, JAZ3ΔJas* and *coi1-30* plants three days after spray inoculation with bacteria at 10⁸ cfu/ml or syringe infiltration with bacteria at 5x10⁵ cfu/ml. Error bars indicate SEM (n = 7). Different letters above columns indicate significant differences compared to their respective WT (Col-7 or Col-0) control samples in each infection condition (Student's t test, p<0.05). ns: no significant. The results are representative of three independent experiments.
   **(D)** Growth of *Pto* DC3000 or a COR-deficient *Pto* DC3000 strain (*Pto* DC3000 *cor*-) on Arabidopsis Col-0, *JAZ2ΔJas* and *coi1-30* plants three days after spray inoculation with bacteria at 10⁸ cfu/ml. Error bars indicate SEM (n = 7). Different letters above columns indicate significant differences compared to WT plants in each infection condition (Student's t test, p<0.001). The results are representative of three independent experiments.

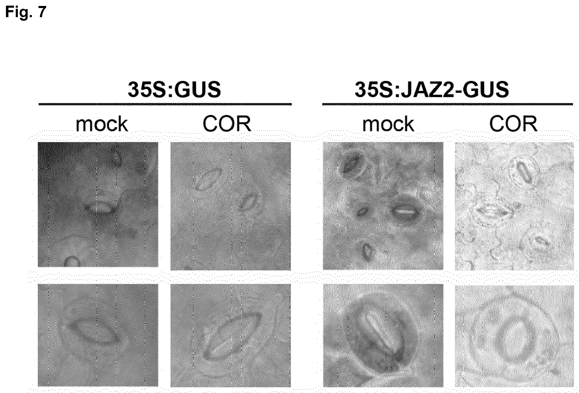

**Figure 7****. COR induced degradation of JAZ2-GUS at stomata.**
   Histochemical GUS activity of 8 days old Arabidopsis transgenic seedlings expressing the GUS reporter gene or *JAZ2-GUS* under the control of the 35S promoter. Seedlings were treated with 50 nM of COR or a mock solution for 3 hours. GUS activity was detected after an overnight staining.

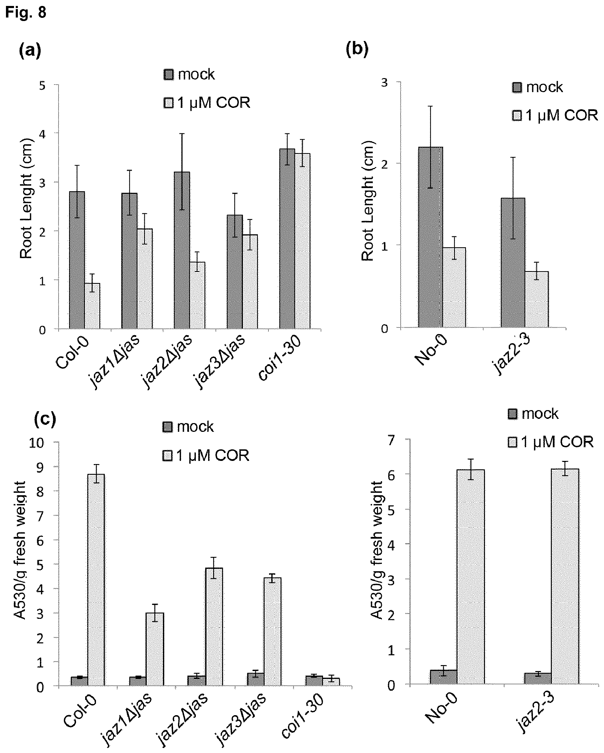

**Figure 8****. Effect of the *JAZ2ΔJas* mutation on root growth inhibition and anthocyanin accumulation by COR.**
   **(A) and (B)** Root growth inhibition assays of 11 days old Arabidopsis seedlings from Col-7, *JAZ1ΔJas,* Col-0, *JAZ2ΔJas, JAZ3ΔJas,* No-0, No-0 *JAZ2-3* and *coi1-30* plants grown in 1 µM COR or mock media. Results shown are the mean ± SD of measurements from 30 seedlings. **(C)** Anthocyanin accumulation of 10 days old Arabidopsis seedlings from Col-7, *JAZ1ΔJas,* Col-0, *JAZ2ΔJas, JAZ3ΔJas,* No-0, No-0 *JAZ2-3* and *coi1-30* plants grown in 1 µM COR or mock media. Results shown are the mean ± SD of measurements from 30 seedlings.

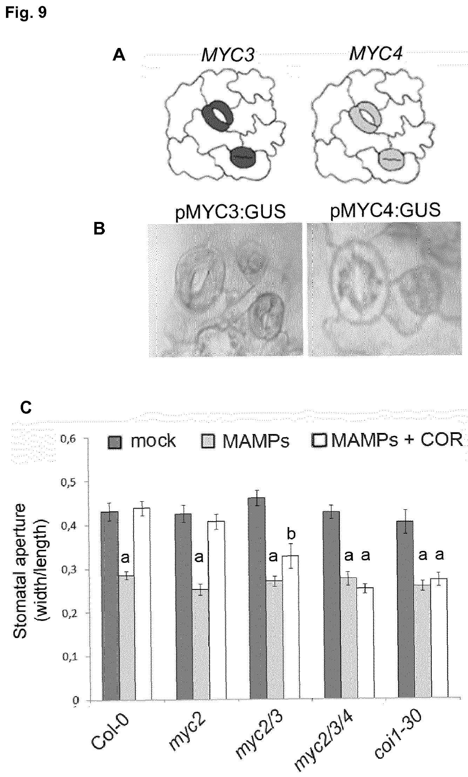

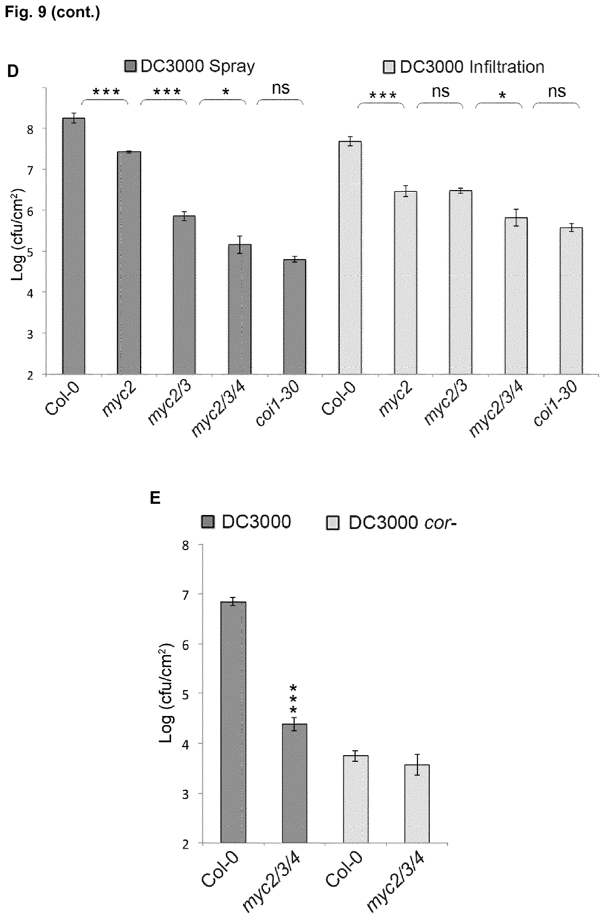

**Figure 9****. MYC2, MYC3 and MYC4 are expressed at stomata and control COR-induced stomatal reopening**
   **(A)** *MYC3* and *MYC4* expression patterns at stomata guard cells. Data from Arabidopsis eFP Browser, http://bbc.botany.utoronto.ca/efp/cgi-bin/efpWeb.cgi **(B)** Histochemical GUS staining of *MYC3* and *MYC4* expression at stomata guard cells. **(C)** Stomatal aperture in Arabidopsis Col-0, *myc2, myc2myc3, myc2myc3myc4* and *coi1-30* leaves measured after 5 hours of incubation with MAMPs (OD₆₀₀=1.5 of crude *Pto* DC3000 extracts), MAMPs plus 2 µM COR or a mock control. Error bars indicate SEM (*n* = 20). Different letters above columns indicate significant differences compared to mock-treated Col-0 control sample (Student's *t* test, p<0.1). The results are representative of three independent experiments. **(D)** Growth *Pto* DC3000 on Arabidopsis Col-0, *myc2, myc2myc3, myc2myc3myc4* and *coi1-30* plants three days after spray inoculation with bacteria at 10⁸ cfu/ml or syringe infiltration with bacteria at 5x10⁵ cfu/ml. Error bars indicate SEM (*n* = 7). Asterisks indicate statistically significant between the indicated Arabidopsis lines (Student's t test, *p<0.05; **p<0.01; ***p<0.001). ns: no significant. The results are representative of two independent experiments. **(E)** Growth of *Pto* DC3000 or a COR-deficient *Pto* DC3000 strain (*Pto* DC3000 *cor*-) on Arabidopsis Col-0 and *myc2myc3myc4* plants three days after spray inoculation with bacteria at 10⁸ cfu/ml. Error bars indicate SEM (*n* = 7). Asterisks indicate statistically significant differences compared to WT plants in each infection condition at *p<0.05; **p<0.01; ***p<0.001). The results are representative of two independent experiments.

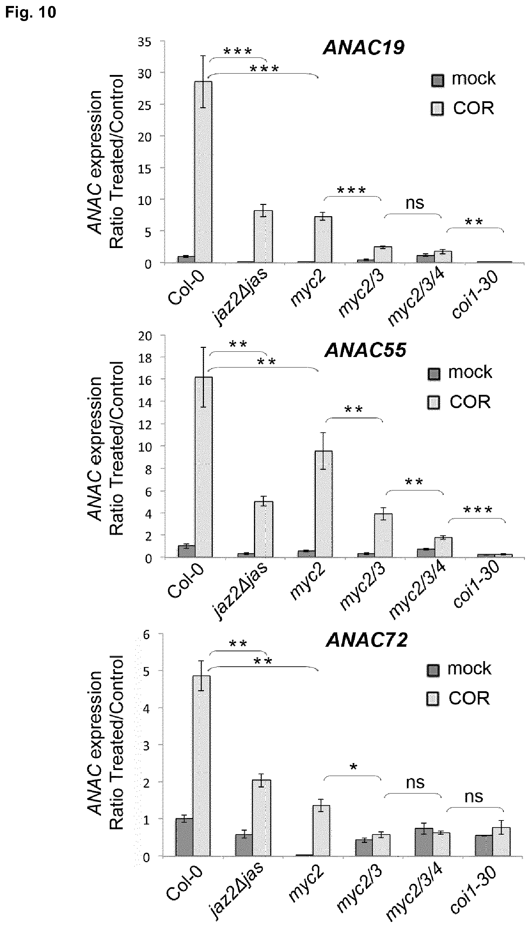

**Figure 10****. MYC2, MYC3, MYC4 and JAZ2 are required for full COR-induction of *ANAC19, ANAC55* and *ANAC72* expression**
   **(A)** Quantitative RT-PCR analysis of *ANAC19, ANAC55* and *ANAC72* expression on *Arabidopsis* Col-0, *myc2, myc2myc3, myc2myc3myc4* and *coi1-30* seedlings induced for 24 hours with 1 µM of COR or a mock solution. The measurements (three technical replicates) represent the ratio of expression levels between each sample and mock treated Col-0. All samples were normalized against the housekeeping gene *At4CT8.* Error bars represent standard deviation (SD). The results are representative of two independent experiments. Asterisks indicate statistically significant differences in the gene induction in each *Arabidopsis* line compared to the previous (Student's *t* test, ***p*<0.01). ns: no significant.

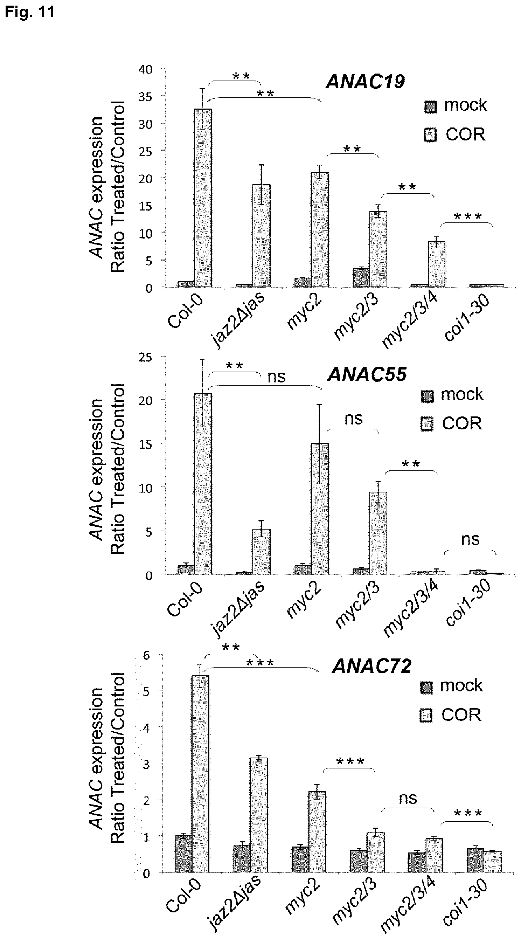

**Figure 11****. MYC2, MYC3, MYC4 and JAZ2 are required for full COR-induction of ANAC19, ANAC55 and ANAC72 expression**
   Quantitative RT-PCR analysis of *ANAC19, ANAC55* and *ANAC72* expression on Arabidopsis Col-0, *myc2, myc2myc3, myc2myc3myc4, JAZ2ΔJas* and *coi1-30* seedlings induced for 20 hours with 1 µM of COR or a mock solution. The measurements (three technical replicates) represent the ratio of expression levels between each sample and mock treated Col-0. All samples were normalized against the housekeeping gene AtACT8. Error bars represent standard deviation (SD). The results are representative of three biologically independent experiments. Asterisks indicate statistically significant differences in the gene induction in each Arabidopsis line compared to the previous as indicated (Student's t test, *p<0.05; **p<0.01; ***p<0.001). ns: no significant.

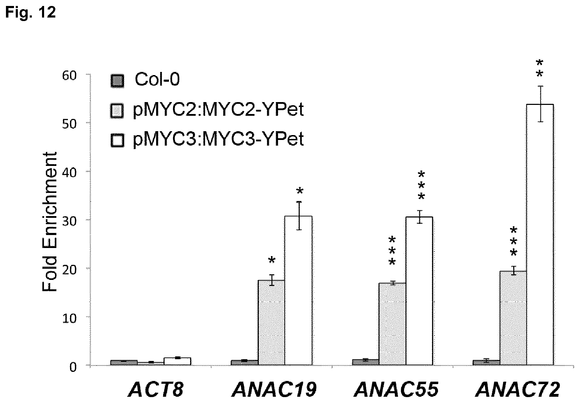

**Figure 12****. MYC2 and MYC3 directly bind to the promoter of *ANAC19, ANAC55* and *ANAC72* genes.**
   *MYC2-YPet* and *MYC3-YPet* transgenic plants under the control of their native promoters were used for ChIP-PCR experiments. Graphic shows fold enrichment of Q-PCR data from ChIP assays with antibody against GFP, which cross-reacts with the GFP derivative YPet, using the ACTIN8 gene as negative control. Error bars represent SD of two technical replicates. Each experiment was repeated twice with similar results. Asterisks indicate statistically significant differences compared to Col-0 for each gene (Student's t test, *p<0.05; **p<0.01; ***p<0.001).

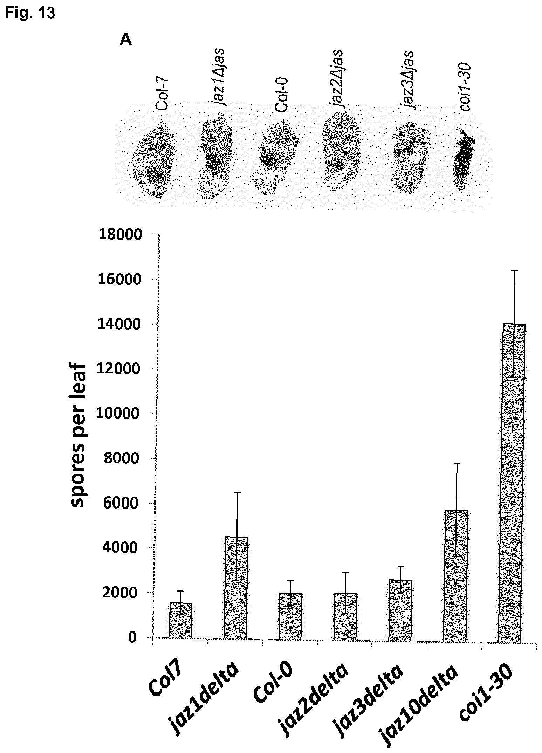

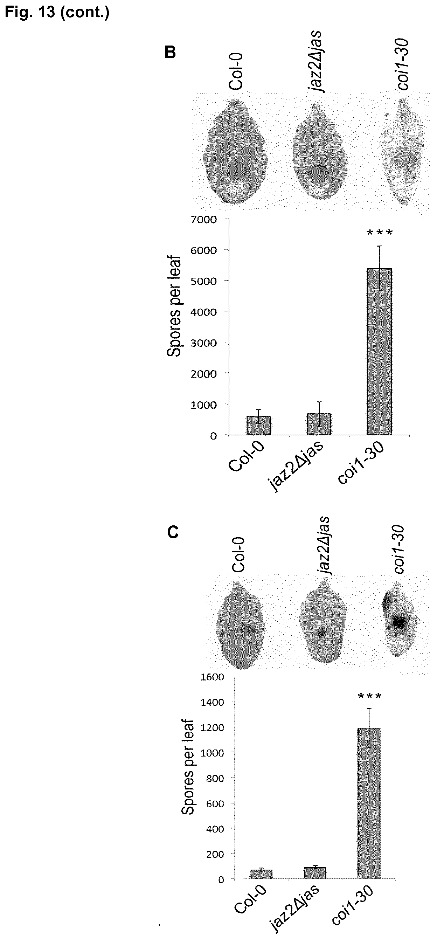

**Figure 13****. *JAZ2ΔJas* mutants retain WT resistance to necrotrophic fungi.**
   **(A)** *B. cinerea* disease symptoms (above) and determination of spore number (below) on Col-7, *JAZ1ΔJas, Col-0, JAZ2ΔJas, JAZ3ΔJas, JAZ10ΔJas* and *coi1-30* plants 6 days after inoculation with 5×10⁶ spores per ml. **(B)** *P. cucumerina* disease symptoms and determination of spore number on Col-0, *JAZ2ΔJas* and *coi1-30* plants 6 days after inoculation with 3×10⁷ spores per ml. **(C)** *A. brassicicola* disease symptoms and determination of spore number on Col-0, *JAZ2ΔJas* and *coi1-30* plants 6 days after inoculation with 1×10⁶ spores per ml. Leaves in A, B and C show representative symptoms of at least two independent experiments. For quantification, three pools per treatment (containing five leaves from five independent plants per pool) were measured. Error bars in A, B and C represent standard deviation (SD). Asterisks indicate statistically significant differences with their respective WT controls (Col-0 or Col-7) (Student's t test, *p<0.05; **p<0.01; ***p<0.001).

### EXAMPLES

### MATERIAL AND METHODS

### Plant Growth Conditions

For *in vitro* assays, seedlings of six to eleven days were growth in MS or Johnson's medium at 21°C under a 16-h-light/8-h-dark cycle. For stomata analysis and bacterial growth assays, *A. thaliana* plants of four to six weeks were grown in controlled environment chambers at an average temperature of 22°C (range 16°C-24°C), with 45%-65% relative humidity under short day conditions (8 h light).

Transgenic Arabidopsis lines expressing *pJAZ:GUS* promoter fusions were obtained by cloning the corresponding *pJAZ* PCR products (pJAZ1 fw caccGTACGTTCCAACTTCTAACC [SEQ ID NO: 29]; pJAZ1 rev CATCTTTAACAATTAAAACTTTCAAACG [SEQ ID NO: 30]; pJAZ2 fw caccGACTAAGAATTTGTTATGAAG [SEQ ID NO: 31]; pJAZ2 rev CATCGTTGAAACCGAAATTGAAATCG [SEQ ID NO: 32]; pJAZ3 fw caccGATCTGGCTCGACGTCGACGAGAAGC [SEQ ID NO: 33]; pJAZ3 rev CATCTATAATAAAGACACAGCCCGC [SEQ ID NO: 34]) into the pENTR™/D-TOPO® system (Invitrogen) and then transferring them to pGWB3. Agrobacterium strain GV3101, containing these constructs, was used to transform Col-0 plants by floral dipping (Clough, S.J. and Bent, A.F. Plant J. 1998, 16(6): 735-743). Several transgenic lines were checked for Kanamycin and Hygromycin resistance and lines carrying one insertion were driven to homozygosis and used for further analysis. *pJAZ2:GUS* in *coi1-30* background was generated by crossing the corresponding parental single homozygous lines. Arabidopsis lines expressing *pMYC3:GUS* and *pMYC4: GUS* promoter fusions, were previously reported (Fernandez-Calvo, P. et al. Plant Cell. 2011, 23(2): 701-715). The lines *JAZ2-3* (RIKEN_13-5433-1) and *JAZ2ΔJas* (GABI_169B06) were obtained from the Nottingham Arabidopsis Stock Centre. The line *JAZ1ΔJas* (SEQ ID NO: 61) was obtained from an Arabidopsis activation tagging population (Weigel, D. et al. Plant Physiol. 2000, 122(4): 1003-1013). Knock out lines *myc2*/*jin1-2* (Lorenzo, O. et al., Plant Cell. 2004, 16(7): 1938-1950; Fernandez-Calvo, P. et al. Plant Cell. 2011; 23(2): 701-715), *myc2*/*myc3, myc2*/*3*/*4* (Fernandez-Calvo, P. et al. Plant Cell. 2011; 23(2): 701-715), *jai3-1* (Chini, A. et al. Nature. 2007; 448(7154)) and *coi1-30* (Yang, D.L. et al. Proc Natl Acad Sci U S A. 2012, 109(19): E1192-1200) were previously described. *coi1-1* was provided by J. Turner.

Transgenic Arabidopsis plants expressing epitope-tagged MYC2 (AT1G32640) and MYC3 (AT5G46760) under control of their native promoters were generated by recombineering. A YPet-3xHA tag was fused to MYC2 and a YPet-6xHis-3xFLAG tag fused to MYC3, immediately before the stop codon and in-frame in both cases. YPet is an enhanced fluorescent tag derived from GFP. Fusions were generated in the transformation competent bacterial artificial chromosome (TAC) clones JAtY76E19 and JAtY69C18, respectively (Alonso, J.M. and Stepanova, A.N. Methods Mol Biol. 2015, 1227: 233-243). These constructs were transformed into wild-type Arabidopsis using floral dip methodology dipping (Clough, S.J. and Bent, A.F. Plant J. 1998, 16(6): 735-743). The expression of tagged MYC2 and MYC3 was confirmed by immunoblotting using an anti-GFP antibody that recognizes YPet. Homozygous transgenic lines exhibiting patterns of resistance marker gene segregation consistent with single site insertion were identified by selection of T2 and T3 generation seedlings on Linsmaier and Skoog medium plates (Caisson labs, USA) containing 15 µg/ml glufosinate ammonium (Sigma-Aldrich, USA). These lines were designated pMYC2:MYC2-YPet and pMYC3:MYC3-YPet, and used to perform ChIP-PCR experiments.

### GUS staining

For *JAZ promoter:GUS* expression experiments, five to seven days-old seedlings were treated with mock solution or 50 µM JA for 2 h and GUS stained as previously described (Fernandez-Calvo, P. et al. Plant Cell. 2011; 23(2): 701-715). For JAZ2 protein stability experiments, seven to nine days-old seedlings were treated with mock solution or 50 nM COR for 3 h. Then, samples were placed in staining solution containing 50 mM phosphate buffer, pH 7, 0.1% (v/v) Triton X-100 (Sigma-Aldrich), 2 mM 5-bromo-4-chloro-3-indolyl b-D glucuronic acid (X-Gluc, Glycosynth), 1 mM potassium-ferrocyanide (Sigma-Aldrich), and 1 mM potassium-ferricyanide (Sigma-Aldrich) and incubated at 37°C overnight. After staining, the tissue was soaked several times in 75% ethanol and kept in 5% glycerol until being photographed with a Leica DMR UV/VIS microscope.

### Bacterial Strain and Bacterial Growth Curves

*Pseudomonas* strains used in this study were *Pseudomonas syringae pv. tomato* (*Pto*) DC3000 and the coronatine-deficient *Pto* DC3000 strain (*Pto* DC3000 COR-) which is a *Pto* DC3000 AK87 mutant that carries mutations in cmaA (coronamic acid A) and cfa6 (coronafacic acid 6).

Bacterial growth assays in Arabidopsis were performed as previously described (Gimenez-Ibanez, S. et al. Plant Signal Behav. 2009, 4(6): 539-541). For spraying infection assays, plants were sprayed with a bacterial suspension containing 10⁸ (cfu)/ml bacteria (OD₆₀₀ = 0.2) with 0.04% Silwet L-77. For bacterial growth assays by syringe infiltration, leaves were syringe infiltrated with a bacterial suspension containing 5x10⁵ (cfu)/ml bacteria (OD₆₀₀ = 0.001). Bacterial growth assays by spray inoculation and syringe infiltration were performed simultaneously and thus, results shown represent comparable experiments. Error bars indicate standard error of the mean (SEM) (n = 7). These experiments were repeated at least three times with similar results, and representative results are shown.

### Stomatal Aperture Measurements

Whole leaves from 4- to 6-week-old Arabidopsis plants were collected and exposed to white light for 1.30 hours while floating in a solution containing 50 mM KCI, 10 µm CaCl₂, and 10 mm MES-KOH pH = 6.1 (opening stomata buffer) with moderate shaking (50 rpm) to induce homogenous stomatal aperture among all Arabidopsis lines to compare. To do the MAMP-induced closure assays, the previous solution was removed and whole leaves were immersed in opening stomata buffer containing *Pto* DC3000 MAMP extracts at OD₆₀₀=1,5 (7,5 ×10⁸ cfu/ml), 2 µM of coronatine or a mock solution. Samples were further incubated under the same previous conditions for 4-5 hours. Then, leaves were rapidly peeled and abaxial epidermal peels were placed on cover slips and observed with a microscope (Leica DMR). Stomatal aperture (width/length) was measured using ImageJ software. Error bars represent standard error of the mean (SEM) (*n* = 20). Stomatal experiments were repeated at least three independent times and representative results are shown.

### Quantitative RT-PCR

Gene expression experiments were performed with RNA extracted from 7 to 10-day-old seedlings grown on liquid MS media that were treated with 1 µM of COR for 5 or 20 h or a mock solution. For experiments monitoring *PR* gene expression, seedlings were treated with DC3000 MAMP extracts at OD₆₀₀=1,5 (7,5 ×10⁸ cfu/ml), 2 µM of coronatine or a mock solution for 5 hours as stomata experiments were performed or 20 hours. Quantitative RT-PCR was performed as it was previously described (Fernandez-Calvo. P. et al. Plant Cell. 2011; 23(2): 701-715). Data analysis shown was done using three technical replicates from one biological sample; similar results were obtained with at least three additional independent biological replicates.

Primers for *ANAC19, ANAC55* and *ANAC72* genes used here were previously described (Zheng, X.Y. et al. Cell Host Microbe. 2012, 11(6): 587-596), and the complete list of primers used in this study are as follows:
*AtANAC19_fw:* 5' -GCATCTCGTCGCTCAG-3' [SEQ ID NO: 35]; and *AtANAC19_rev* 5' - CTCGACTTCCTCCTCCG-3' ; [SEQ ID NO: 36]; *AtANAC55_fw:* 5' - GCGCTGCCTCATAGTC-3' [SEQ ID NO: 37] and *AtANAC55*_*rev* 5' -CGAGGAATCCCCTCAGT-3' [SEQ ID NO: 38]; *AtANAC72_fw:* 5' - TGGGTGTTGTGTCGAAT-3' [SEQ ID NO: 39] and *AtANAC72*_*rev* 5' - ATCGTAACCACCGTAACT-3' [SEQ ID NO: 40]; *AtACTIN8_fw:* 5' -CCAGTGGTCGTACAACCGGTA-3' [SEQ ID NO: 41] and *AtACTIN8_rev:* 5' -TAGTTCTTTTCGATGGAGGAGCTG-3' [SEQ ID NO: 42]; *AtJAZ2* Full Length Forward (*AtJAZ2* FL_F), 5' - CGAGTGTTGGGACTTCTCTG -3 [SEQ ID NO: 43]; *AtJAZ2* Full Length Reverse (*AtJAZ2* FL_R), 5' - GCTCTTCTTGCAATCGGGAGT-3' [SEQ ID NO: 44]; *AtJAZ2-3* Forward (*AtJAZ2-*3_F), 5' -TGGATTCTTCTGCTGGTCAA -3' [SEQ ID NO: 45]; *AtJAZ2ΔJas* Forward (*JAZ2ΔJas_F*), 5' - TGCTTCTAGCCCAAATCCTG-3' [SEQ ID NO: 46]; *AtJAZ2ΔJas* Reverse (*JAZ2ΔJas_R*), 5' - TCTTTGGTCCCAGAGGAAGA-3' [SEQ ID NO: 47]; *AtPR1*, 5' -GTGGGTTAGCGAGAAGGCTA-3' [SEQ ID NO: 48] and 5' -ACTTTGGCACATCCGAGTCT-3' [SEQ ID NO: 49]; *AtPR2,* 5' - GTCTGAATCAAGGAGCTTAGCC-3' [SEQ ID NO: 50] and 5' - CCGTAGCATACTCCGATTTGT-3' [SEQ ID NO: 51].

### Root growth and anthocyanin measurements

Root growth, chlorophyll and anthocyanin measurements were performed as previously described (Fonseca, S. et al. PLoS One. 2014, 9(1): e86182.). In both cases, 20 to 30 seedlings were measured seven to eleven days after germination in presence or absence of 1 µM of COR or a mock control. Values represent mean and standard deviation (SD). Results are representative of at least two independent experiments.

### ChIP-PCR

For pMYC2:MYC2-YPet and pMYC3:MYC3-YPet ChIP, ten-days-old seedlings were treated with1 µM of COR for 4 h. ChIP was performed as previously described (Busch, W. et al. Dev Cell. 2010, 18(5): 849-861) using an anti-GFP that cross-reacts with YPet and the samples were analyzed by qPCR. Input samples were first used to normalize the results. Fold difference was then calculated by taking ratios between normalized results from the probes (ChIP with antibody against GFP) and the corresponding control (ChIP with no antibody). Finally, the fold enrichment was calculated as the ratio between non-transgenic Col-0 plants and from pMYC2:MYC2-YPet or pMYC3:MYC3-YPet plants.

Primers were designed to amplify positions of putative MYC2 and MYC3 binding sites in *ANAC019, ANAC055* and *ANAC072* promoters at -991, -625 and -776 base pairs from transcription start site were, respectively. The primers used for qPCR are as follows:
*AtANAC19* G-box (*At ANAC19* G-box F), 5' -AGCAGCTACTACGAGTTGTGT-3' [SEQ ID NO: 52]; *AtANAC19* G-box (*At ANAC19* G-box R), 5' - CGTGTCCACGTGTCTATCGT-3' [SEQ ID NO: 53]; *AtANAC55* G-box (*At ANAC55* G-box F), 5' -TGTGTCGGCTTGTGGTAGTT-3' [SEQ ID NO: 54]; *AtANAC55* G-box *(At ANAC55* G-box R), 5' -GGGATGAGTTCACTGGATGGT-3' [SEQ ID NO: 55]; *AtANAC72* G-box *(At ANAC72* G-box F), 5' - TGCAATCACTCAGCGGACTT-3' [SEQ ID NO: 56]; *AtANAC72* G-box *(At ANAC72* G-box R), 5' -GGCCGACCTTATCGATGTGT-3' [SEQ ID NO: 57]; *AtACTIN8* CHIP, (*AtACTIN8* ChIP F) 5' -GACTCAGATCATGTTTGAGACCTTT-3' [SEQ ID NO: 58] and (*AtACTIN8* ChIP R) 5' -ACCGGTTGTACGACCACTGG-3' [SEQ ID NO: 59].

### Botrytis cinerea infection assays

*B. cinerea* infection assays were performed as previously described (Monte, I. et al. Nat Chem Biol. 2014, 10(8): 671-676). Briefly five-week-old Arabidopsis plants were inoculated with 20 ul of a suspension of 5 ×10⁶ spores/ml PDB (Difco). At least 15 leaves (3 leaves per plant) were inoculated per treatment. Disease symptoms were scored 6 days after inoculation. Spores were quantified in a hemocytometer with a light microscope (Leica DMR UV/VIS). Five inoculated leaves of five different plants were pooled for each replicate. Three independent replicates were measured for each treatment. This experiment was repeated twice with similar results.

*P. cucumerina* and *A. brassicicola* infections assays were performed as previously described for *B. cinerea* but inoculating each leaf with 20 µl of a suspension of 3 × 10⁷ (*P. cucumerina*) or 1 ×10⁶ (*A. brassicicola*) spores/ml PDB. Disease symptoms and spores were quantified as previously described for *B. cinerea*

### Example 1. JAZ2 gene is expressed in stomata guard cells.

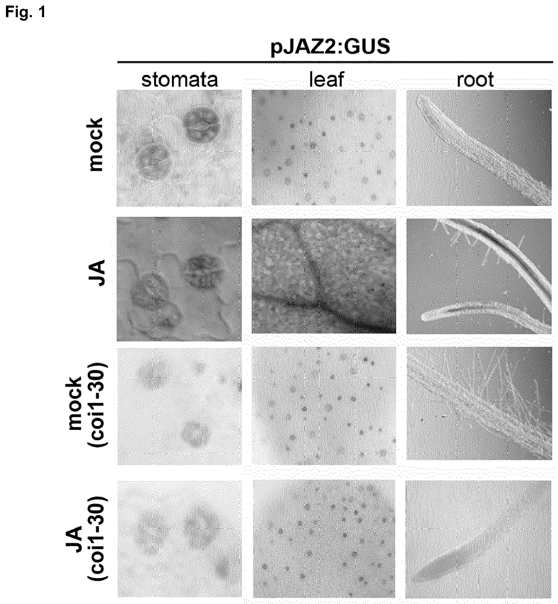

To identify *JAZ* genes specifically expressed at stomata, inventors produced transgenic Arabidopsis lines harboring a promoter construct covering around 2 Kb upstream of the ATG of each JAZ fused to the β-glucuronidase (GUS) reporter gene. It was successfully obtained stable transgenic plants expressing the corresponding JAZ promoter:GUS fusions for seven out of the twelve *JAZ* genes (JAZ1; AT1G19180, JAZ2; AT1G74950, JAZ3; AT3G17860, JAZ5; AT1G17380, JAZ6; AT1G72450, JAZ9; AT1G70700 and JAZ12; AT5G20900). Detailed examination of GUS expression driven by JAZ regulatory sequences at the stomata revealed that JAZ2 was the only one among these JAZs expressed in guard cells (Fig. 1). In basal conditions, expression of JAZ2 was exclusive of guard cells, but JA treatment induced it in roots and mesophyll cells of young seedlings, which partially masked the guard cell signal. Histochemical analyses of pJAZ2:GUS transgenics into the coi1-30 background (coi1-30-null mutant) revealed that basal expression of JAZ2 at the stomata occurs in a COI1-independent manner (Fig. 1). However, COI1 is required for JA-responsiveness of JAZ2 in mesophyll and root cells.

### Example 2. JAZ2 regulates stomata dynamics during bacterial pathogenesis.

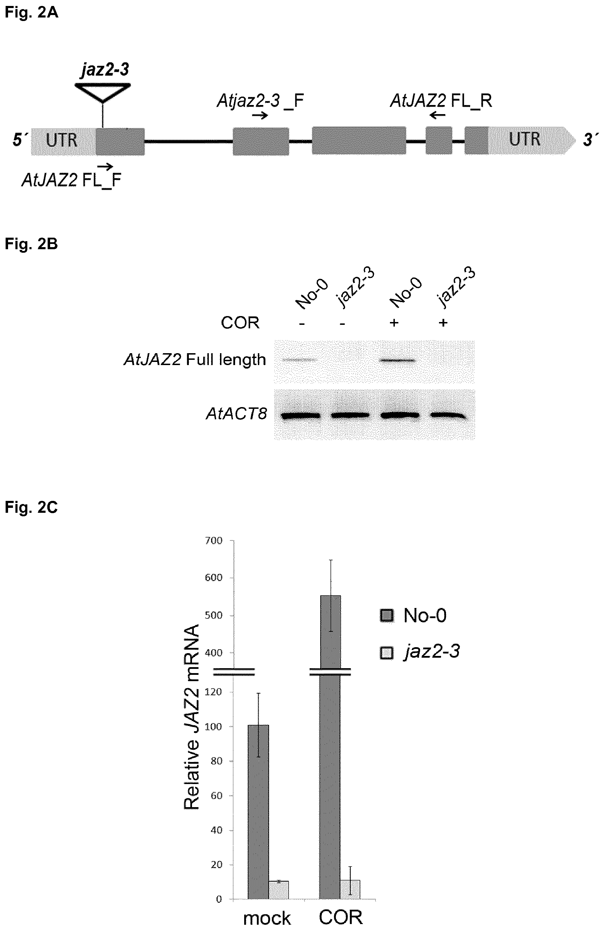

To test whether JAZ2 function as a regulator of stomatal dynamics during bacterial invasion, it was first obtained an *Arabidopsis* transposon insertion mutant, designated *JAZ2-3* (SEQ ID NO: 12), in the accession Nossen (No-0), and selected homozygous plants (Fig. 2). Gene expression analyses supported that this mutant is a knock-out, or at least a knock-down, since it does not express the full-length *JAZ2* gene, and expresses very low levels of truncated mRNA 3' downstream the insertion (Fig. 2).

We next analyzed the ability of *JAZ2-3* to close stomata upon microbial perception and reopen it in the presence of COR. MAMPs in crude boiled *Pto* DC3000 bacterial extracts induce stomata closing (Kunze, G. et al. Plant Cell. 2004; 16(12): 3496-3507; Gimenez-Ibanez, S. et al. Plant Signal Behav. 2009, 4(6): 539-54), whereas addition of COR to these extracts promote stomata reopening (Melotto, M. et al. Cell. 2006; 126(5): 969-980). Thus, whole leaves were incubated with bacterial extracts (MAMPs), MAMPs plus COR or a mock solution as a control. The crude *Pto* DC3000 MAMP extracts were prepared as previously described (Kunze, G. et al. Plant Cell. 2004; 16(12): 3496-3507; Gimenez-Ibanez, S. et al. Plant Signal Behav. 2009, 4(6): 539-54). Briefly, *Pto* DC3000 was grown in LB-medium at 28°C on a rotary shaker until OD₆₀₀∼0.6-1.0. Bacteria were harvested by centrifugation, washed and resuspended in water (20 to 30% cells [fresh weight]/volume). Crude bacterial extracts were prepared by boiling the bacterial suspensions for 10 minutes and kept as cell containing *Pto* DC3000 MAMP crude extracts at -20 degrees. Typically, *Pto* DC3000 MAMPs were used at optical density at 600 nm OD₆₀₀=1,5 (7,5 ×10⁸ cfu/ml) for incubation with leaves (Stomatal Aperture Measurements) or Arabidopsis seedlings (Quantitative RT-PCR). COR was purchased from Sigma-Aldrich and dissolved in EtOH. The results show that MAMPs induced stomatal closure in WT plants and *coi1-30* mutants (coi1-30-null mutant). However, the single *JAZ2-3* mutant comprising the transposon of SEQ ID NO: 12, obtained as it is mentioned above, was partially impaired in MAMP-induced stomata closing (Fig. 3A). Incubation of leaves with MAMPs and COR, simultaneously, triggered COR-induced stomata reopening in *JAZ2-3* and WT plants, but not in *coi1-30* (coil-30-null mutant) (Fig. 3A). These results support previous observations indicating that COR-induced reopening of stomata is dependent on COR perception through the co-receptor COI1 (Melotto, M., et al. Cell. 2006; 126(5): 969-980), and suggests that JAZ2 plays a role in modulating stomata closure after bacterial perception.

Pathogen-induced stomatal closure in Arabidopsis depends on SA biosynthesis and signaling pathways (Melotto, M., et al. Cell. 2006; 126(5): 969-980; Zeng, W. and He, S.Y. Plant Physiol 2010; 153(3): 1188-1198; Zeng, W. et al. PLoS Pathog. 2011; 7(10): e1002291; Arnaud, D. and Hwang, I. Mol Plant 2015; 8(4): 566-581). Thus, it was evaluated the ability of WT and the single *JAZ2-3* mutant to activate SA signaling upon MAMP perception by monitoring the expression of the SA marker genes *PR1* and PR2. MAMPs induced significantly *PR1* and PR2 expression in WT plants after 5 or 20 hours of incubation (Fig. 3B). In contrast, MAMP induced expression of these genes was strongly compromised in the single *JAZ2-3* mutant compared to its control mock treatment. This suggests that the compromised ability of plants lacking *JAZ2* to trigger MAMP induced stomatal closure might be the consequence of a defective activation of SA signaling pathway upon pathogen perception.

In addition to induce stomatal reopening, COR is also essential for the bacteria to overcome the mesophyll cell-based defenses occurring in the apoplast (Cui, J. et al. Proc Natl Acad Sci USA. 2005; 102(5): 1791-1796; Laurie-Berry, N. et al. Mol Plant Microbe Interact. 2006; 19(7): 789-800; Zeng, W., et al. PLoS Pathog. 2011; 7(10): e1002291; Zheng, X. Y. et al. Cell Host Microbe. 2012; 11(6): 587-596). To differentiate the contribution of JAZ2 to bacterial defense by either regulating stomata apertures and/or mesophyll cell-based defences in the apoplast, it was compared bacterial replication of *P. syringae* pv. *tomato* DC3000 (*Pto* DC3000) on WT (No-0) and *JAZ2-3 Arabidopsis* plants infected by spray inoculation or siringe infiltration (Zipfel, C. et al. Nature. 2004; 428(6984): 764-767). The spray inoculation technique mimics natural infection conditions and is one of the most sensitive techniques to assess plant susceptibility to bacterial pathogens (Zipfel, C. et al. Nature. 2004; 428(6984): 764-767). In contrast, the syringe infiltration bypasses the early stomatal level of regulation measuring mainly apoplastic cell-based defences. Three days post-spray inoculation bacterial titers in *JAZ2-3* plants were significantly higher than those in its respective WT control. However, the titers were similar in *JAZ2-3* and WT after infiltration (Fig. 3C). These results indicate that *JAZ2* negatively regulates disease resistance to *Pseudomonas* bacteria and support a major role for *JAZ2* in early penetration stages of the bacteria through the stomata compared to the weak role of *JAZ2* in apoplastic cell-based defenses.

### Example 3. Dominant negative JAZ2ΔJas mutants are impaired in COR-induced stomata re-opening and are resistance to P.syringae infections.

To study the specific function of *JAZ2* at stomata analyses of dominant-negative *JAZ* variants were also performed. Truncated JAZ forms lacking the C-terminal Jas domain (JAZΔJas) are resistant to COI1-dependent degradation and behave as constitutive active repressors, blocking the activity of TFs and conferring JA-insensitivity (Chini, A. et al. Nature. 2007; 448(7154): 666-671; Katsir, L. et al. Proc Natl Acad Sci USA. 2008; 105(19): 7100-7105; Sheard, L. B. et al. Nature. 2010; 468(7322): 400-405; Moreno, J. E. et al. Plant Physiol. 2013; 162(2): 1006-1017). To further evaluate the effect of these gain-of-function repressors in stomatal dynamics inventors searched for *Arabidopsis* T-DNA insertion mutants that would result in JAZ2ΔJas forms under the control of its natural genomic context. It was identified a T-DNA line (GABI collection) that contained an insertion in the third exon (Fig. 4A and B), and therefore, translated *JAZ2* into an aberrant protein lacking the C-terminal Jas domain, this mutant was designated as *JAZ2ΔJas* (SEQ ID NO: 10).

As a control for specificity, it was also included in these analyses other *JAZΔJas* mutants from *JAZ* genes not expressed at stomata. Thus, was used the previously described *jai3-1* dominant mutant (hereafter named *JAZ3ΔJas,* SEQ ID NO: 14 for the cDNA and SEQ ID NO: 15 for the aminoacid sequence) (Chini, A. et al. Nature. 2007; 448(7154): 666-671). *JAZ3* is prevalently expressed in roots, both in basal and JA induced conditions, but is not expressed at stomata (Fig. 5B). See SEQ ID NO: 13 for the promoter of JAZ3. Additionally, a newly identified *JAZ1ΔJas* (SEQ ID NO: 60 for the cDNA and SEQ ID NO: 61 for the aminoacid sequence) in the Arabidopsis accession Col-7 (Fig. 4C) also behaves as a dominant mutant. JAZ1 was also expressed in roots in basal conditions whereas JA treatment strongly induced JAZ1 in roots and mesophyll cells (Fig. 5A). Therefore, JAZ1 (Fig. 5A), JAZ2 (Fig. 1) and JAZ3 (Fig. 5B) have different expression patterns, which suggest that they may have diverse functional specificities.

To asses the effect of these differernt mutations in stomatal and apoplastic immunity, whole leaves of *JAZ1ΔJas* (SEQ ID NO: 61), *JAZ2ΔJas* (SEQ ID NO: 10) and *JAZ3ΔJas* (SEQ ID NO: 15) mutants, and their respective WT backgrounds were incubated with MAMPs, MAMPs plus COR or a mock control, as described above. Similar to WT and *coi1-30* plants, the dominant-negative version of JAZ1 (*JAZ1ΔJas*), JAZ2 (*JAZ2ΔJas*) and JAZ3 (*JAZ3ΔJas*) closed stomata when incubated with MAMPs, indicating that these repressive truncated JAZs forms do not compromise stomata closing upon microbial perception (Fig. 6A). When leaves were incubated with MAMPs and COR simultaneously, WT, *JAZ1ΔJas* and *JAZ3ΔJas* Arabidopsis plants induced COR-mediated stomatal reopening as expected (Fig. 6A). However, similar to *coi1-30, JAZ2ΔJas* mutants were fully impaired in COR-mediated stomatal reopening. This indicates that JAZ2 regulates stomata closure and that COR-induced stomata reopening require inhibition of JAZ2.

It was next compared bacterial replication of *Pto* DC3000 on *Arabidopsis* WT (Col-0), *JAZ1ΔJas, JAZ2ΔJas, JAZ3ΔJas* and *coi1-30* plants infected by spray inoculation or syringe infiltration. Infections with sprayed *Pto* DC3000 bacteria showed similar symptom development on WT and *JAZ3ΔJas* plants (Fig. 6B). In contrast, *JAZ1ΔJas* and *JAZ2ΔJas* were remarkably more resistant than WT and resembled *coi1-30* mutants, with very few chlorotic symptoms typical of *Pseudomonas* infections (Fig. 6B). Plant symptomatology correlated well with bacterial titers. Similar to *coi1-30* plants, *JAZ1ΔJas* and *JAZ2ΔJas* leaves sprayed with *Pto* DC3000 contained remarkably lower bacterial titers than WT, whereas bacterial counts in *JAZ3ΔJas* plants were significantly higher and close to WT levels (Fig. 6C). Still, bacterial levels in *JAZ2ΔJas* leaves were always slightly higher than *coi1-30* plants. There were next performed *Pto* DC3000 infection assays by syringe infiltration of *Pto* DC3000 into the apoplast. *JAZ3ΔJas* leaves contained similar bacterial counts as those observed in WT (Col-0) plants when *Pto* DC3000 bacteria was sprayed onto the leave, indicating that JAZ3 does not play a major role in regulating COR-induced stomatal aperture nor apoplastic defense responses in aerial tissues (Fig. 6C). In contrast, *JAZ1ΔJas* leaves contained still lower bacterial counts compared to WT plants when *Pto* DC3000 bacteria were infiltrated onto the leaf, suggesting that JAZ1 plays a major role in the regulation of apoplastic defense responses (Fig. 6C). Remarkably, disease susceptibility could be restored in the *JAZ2ΔJas* mutant when bypassing stomata regulation through syringe injection of bacteria (Fig. 6C). Indeed, differences in bacterial growth observed when *Pto* DC3000 bacteria was sprayed were significantly diminished when the same bacteria was injected into the leave, supporting the idea that JAZ2 functions during the early penetration process of *Pto* DC3000 bacteria by specifically regulating stomatal aperture. Still, *JAZ2ΔJas* leaves typically contained slightly less bacterial counts than WT plants which reflect a minor effect of JAZ2 on aploplastic defense.

To further support the idea that COR contributes to promote bacterial pathogenicity by targeting JAZ2, there were sprayed *Pto* DC3000 or the COR-deficient *Pto* DC3000 *cor-* bacteria onto WT, *JAZ2ΔJas* and *coi1-30* plants. As previously observed, three days after spray with *Pto* DC3000, WT leaves contained higher bacterial titers than *JAZ2ΔJas* and *coi1-30* mutants (Fig. 6D). In contrast, all WT, *JAZ2ΔJas* and *coi1-30* plants exhibited similar levels of bacterial growth when the COR-deficient *Pto* DC3000 *cor-* was sprayed onto the leaves (Fig. 6D). These results indicate again that the virulence effect of COR during the bacterial infective process requires elimination of JAZ2, and therefore, cannot be exerted in this constitutively active (stable) variant of JAZ2, which is resistant to degradation. Supporting this, further experiments to determine JAZ2 protein stability in the presence of COR at stomata indicated that concentrations as low as 50 nM of COR induced the rapid degradation of JAZ2 protein at guard cells in transgenic Arabidopsis plants ectopically expressing *JAZ2* fused to the GUS reported gene (Fig. 7). Altogether, these data support a prominent role of JAZ2 in stomatal movement regulation during the infection process of phytopathogenic *Pseudomonas* bacteria.

### Example 4. JAZ2 has a minor role in JA-responses outside guard cells.

To further support the specificity of JAZ2 in guard cell regulation, it was analyzed other typical JA-regulated responses, such as root-growth inhibition and anthocyanin accumulation. As shown in Fig. 8, root-growth inhibition in response to COR was unaffected in either *JAZ2-3* or *JAZ2ΔJas* plants. In contrast, *JAZ1ΔJas* and *JAZ3ΔJas* were markedly insensitive to COR, which is consistent with their expression patterns (Fig. 8A and B). Regarding anthocyanin accumulation, *JAZ1ΔJas* plants were severely compromised in their ability to accumulate anthocyanin in response to COR, whereas *JAZ2ΔJas* and *JAZ3ΔJas* dominant JAZ versions showed a milder decrease in anthocyanin accumulation compare to *JAZ1ΔJas* plants (Fig. 8C). *JAZ2-3* mutant plants showed completely normal anthocyanin accumulation in response to COR (Fig. 8C).

### Example 5. MYC2, MYC3 and MYC4 regulate redundantly COR-mediated stomatal reopening.

COR-induced stomatal reopening and apoplastic defense is achieved through direct activation of *ANAC19, ANAC55* and *ANAC72* by the TF MYC2 (Zheng, X. Y. et al. Cell Host Microbe. 2012; 11(6): 587-596). However, COR-induced *NAC* induction is not completely abolished in a *myc2* mutant (Zheng, X. Y. et al. Cell Host Microbe. 2012; 11(6): 587-596), suggesting that additional TFs should play a redundant function with MYC2 in regulating COR-induced stomatal reopening. MYC3 and MYC4 are also targets of JAZ repressors, JAZ2 among others, and regulate redundantly with MYC2 some JA-Ile-dependent responses, including pathogen resistance (Cheng, Z. et al. Mol Plant. 2011; 4(2): 279-288; Fernandez-Calvo, P. et al. Plant Cell. 2011; 23(2): 701-715; Niu, Y. et al. J Exp Bot. 2011; 62(6): 2143-2154; Moreno, J. E. et al. Plant Physiol. 2013; 162(2): 1006-1017). To investigate whether MYC3 and/or MYC4 could play a redundant role with MYC2 in regulating COR-mediated stomata reopening, inventors first examined the tissue-specific expression patterns of these TFs. Gene expression data in public databases (like http://bbc.botany.utoronto.ca/efp) indicated that MYC3 and MYC4 are expressed in mature guard cells, although the expression of MYC4 was much lower (Fig. 9A). Promoter-GUS fusion assays of MYC3 and MYC4 confirmed these data (Fig. 9B).

It was next analyzed the ability of single *myc2*, double *myc2myc3* and triple *myc2myc3myc4* mutants to induce COR-mediated stomatal reopening. It was included in these experiments *coi1-30* as a negative control. As expected, all mutants and WT plants closed stomata when incubated with MAMP extracts (Fig. 9C). However, in the presence of MAMPs and COR, *myc2myc3myc4* mutants could not reopen stomata in a similar fashion as *coi1-30* (Fig. 9C). *myc2myc3* mutants showed an intermediate phenotype, whereas the single *myc2* mutant induced COR-mediatd stomatal reopening similar to WT plants. Altogether, these results indicate that depletion of these three MYCs is sufficient to render plants fully insensitive to COR in terms of stomata reopening and that MYC2, MYC3 and MYC4 play redundant roles in controlling COR-induced stomata reopening.

COI1 regulates both apoplastic defences and stomatal re-opening (Melotto, M., et al. Cell. 2006; 126(5): 969-980; Zheng, X. Y. et al. Cell Host Microbe. 2012; 11 (6): 587-596). Bacterial growth after infiltration or spray inoculation was very similar in *myc2myc3myc4* and *coi1-30*, further supporting that these MYCs also regulate both processes (Fig. 9D). Consistent with a redundant function, the single *myc2* and double *myc2myc3* mutants showed an intermediate phenotype between WT and the triple *myc2myc3myc4* when bacteria were spray inoculated onte the leaves or directly infiltrated into the apoplast (Fig. 9D). These results are consistent with the effects on *myc2, myc3* and *myc4* mutants on stomatal aperture. Moreover, it was also analysed the extent to which COR contributes to promotion of bacterial pathogenicity through these MYCs. When COR-deficient *Pto* DC3000 COR-bacteria was sprayed onto WT and *myc2myc3myc4* plants, both exhibited rather similar levels of bacterial growth compared to the almost three log (cfu/cm²) difference that it was typically observed when *Pto* DC3000 was sprayed onto the same plants (Fig. 9E), supporting the notion that the virulence effect of COR is mediated by these three MYCs.

Altogether, these results suggest that MYC2, MYC3 and MYC4 act redundantly controlling both COR-induced stomata reopening and apoplastic defenses.

### Example 6. MYC2, MYC3, MYC4 and JAZ2 control COR-dependent expression of ANAC19, ANAC55 and ANAC72.

Next it was evaluated whether MYCs regulate *ANAC* gene expression. Inventors monitored expression of *ANAC19, ANAC55* and *ANAC72* in *myc* mutant seedlings, *such as, JAZ2ΔJas, myc2, myc2myc3* and *myc2myc3myc4* mutants treated for 5 and 20 hours with COR or a mock solution. As shown in Fig. 10 and 11, COR-induced expression of *ANAC19, ANAC55* and *ANAC72* was severely compromised in the *JAZ2ΔJas, myc2* and *myc2myc3* mutants and almost completely abolished in the triple *myc2myc3mcy4* mutant. These results indicate that MYC3 and MYC4 are required for full COR-dependent induction of *ANAC19, ANAC55* and *ANAC72* and act additively with MYC2. Moreover, the results also support that JAZ2 is a repressor of *ANAC* gene expression mediated by MYCs in response to COR. These results are consistent with the phenotypic data showing increased bacterial susceptibility and impaired stomatal aperture in the *myc* mutants (Fig. 9). Moreover, they suggest that these three MYCs are sufficient to explain almost all COI1-dependent regulation of COR-induced stomatal reopening and apoplastic defences.

MYC2 can bind the promoter of *ANAC* genes and regulate their expression when overexpressed in transgenic plants. Since overexpression might be prone to artifacts, the inventors tested whether MYCs regulated by their native promoters could bind directly to the promoter of *ANAC* genes. Thus, we performed chromatin immuno-precipitation (ChIP)-PCR experiments using transgenic Arabidopsis plants expressing a MYC2-YPet-3xHA or MYC3-YPet-6xHis-3xFLAG fusion protein from their native genomic context. These were generated using recombineering methodology to insert seamlessly tags immediately before each gene stop codons. The results of ChIP-PCR showed that MYC2 and MYC3 bind efficiently to the promoters of *ANAC19, ANAC55* and *ANAC72* genes but not to control gene ACT8 (Fig. 12). These results indicate that both MYC2 and MYC3 activate the expression of the three *ANACs* by direct interaction with their promoters under their natural genomic contexts.

Altogether, our data indicate that COR-COI1-JAZ2-MYC2/3/4-NAC19/55/72 forms a signaling module controlling stomatal responses during the invasive process of phytopathogenic *Pseudomonas syringae,* whereas other JAZs would likely form such modules to regulate apoplastic defenses.

### Example 7. The JAZ2ΔJas mutant shows unaltered levels of resistance against the necrotrophic pathogens

JA and SA defense pathways generally antagonize each other and thus, strategies to enhance apoplastic defenses against necrotrophs often lead to increased susceptibility to biotrophs, and vice versa (Grant, M. and Lamb, C. Curr Opin Plant Biol. 2006; 9(4):414-20). The cell specific JAZ2 function at guard cells implies that *JAZ2ΔJas* mutation should not compromise apoplastic defences to necrotrophs which invade the plant tissue by active penetration through enzymes and/or appressoria-like structures that establish a primary lesion through which the pathogen can penetrate into the host surface. To test this, it was measured susceptibility of WT, *JAZIΔJas, JAZ2ΔJas, JAZ3ΔJas* and *coi1-30* plants to the necrotrophic fungi *B. cinerea.* Since JAZ1 is expressed in leaves and roots (Fig. 5), JAZ3 is preferentially expressed in roots (Fig. 5) and COI1 is widely expressed in most tissues, therefore *JAZ1ΔJas, JAZ3ΔJas* and *coi1-30* mutants are appropriate controls. Consistent with published data, six days after *B. cinerea* infection *coi1-30 Arabidopsis* plants showed enhanced fungal symptoms and increased spore production compared to control plants (Fig. 13). Consistent with JAZ1 expression in leaves, *JAZ1ΔJas and JAZ10ΔJas* (Moreno, JE. et al. Plant Phys. 2013; 162: 1006-1017. Line 14.4 *pJAZ10:HA-JAZ10.4*) also supported significantly higher number of spores compared to WT plants despite it was not comparable to the levels of susceptibility of *coi1-30* plants. In contrast, *JAZ2ΔJas* and *JAZ3ΔJas* behave similarly to WT in terms of fungal symptoms and spore production (Fig. 13A), indicating that the *JAZ2ΔJas* mutation does not alter apoplastic susceptibility to the necrotrophic fungus *B. cinerea,* but not other JAZΔJas forms such as *JAZ1ΔJas* and *JAZ10ΔJas*. Indeed, it was often observed that the number of spores in *JAZ2ΔJas* leaves were even lower than in WT, suggesting that *B. cinerea* may also use stomata to favor fungal invasion.

Analyses of other necrotrophic fungi such as *Plectosphaerella cucumerina* (Fig. 13B) and *Alternaria brassicicola* (Fig. 13C) gave similar results, indicating that *JAZ2ΔJas* retains WT levels of resistance to a broad range of necrotrophs. Six days after *P. cucumerina* and *A. brassicicola* infection *coi1-30* plants showed severe fungal symptoms and increased spore production compared to WT plants. In contrast, *JAZ2ΔJas* resembled WT plants with similar symptoms and content of spores in leaves (Fig. 13 B,C).

These results further support a key role of JAZ2 in regulating stomatal dynamics, but only a minor role in apoplastic defence responses. Moreover, these results suggest novel strategies for crop protection by manipulating JA/COR-dependent signaling events at the entry ports of specific microbes through the expression of JAZΔJas forms at guard cells. These new strategies will increase resistance to biotrophs without affecting susceptibility to other general pathogens due to the well-known antagonism between JA and SA.

By the improved understanding of the the SA and JA defense pathways that has been achieved by the inventors, the present invention enhances the development of methods for improving the tolerance of plants to biotrophic pathogens (i.e. P. *syringae)* without enhancing susceptibility to necrotrophs (i.e. *B. cinerea, P. cucumerina*, *A. brassiccicola*), as well as for developing easier and more efficient methods for identifying pathogen-tolerant plants. The plants that can be developed using the methods of the present invention have a broad-spectrum resistance to biothrophs but without compromising their resistance to necrotrophs, and provide more and better plant production and consecuently plant products for market in both developed and underdeveloped countries.

As conclusion, the results showed in the present invention show that phytopathogenic *Pseudomonas* produces COR to hijack a COI1-JAZ2-MYC2/3/4-NAC19/55/72 signaling module controlling stomatal responses during the invasive process. Cell-specific expression of JAZ2 at guard cells state that JAZ2 regulates stomatal dynamics during bacterial invasion. Loss- and gain-of-function analyses using *JAZ2-3* and *JAZ2ΔJas* mutants after infiltration or spray inoculation confirmed these results, and supported the fact that JAZ2 primarily functions at the guard cells. The gain-of-function *JAZ2ΔJas* allele blocked stomatal reopening and increased resistance to *Pseudomonas,* whereas the loss-of-function *JAZ2-3* was partially impaired in stomatal closure and more susceptible to the bacteria. This partial phenotype of *JAZ2-3* in stomatal closure could be due to redundancy among JAZ proteins. In fact, COR can still be perceived in the *JAZ2-3* mutant at the stomatal guard cells suggesting that in these cells other JAZ proteins should form co-receptor complexes with COI1, in addition to JAZ2. The inventors succeed in analyzing the expression patterns of seven JAZs only, and therefore, a role for the remaining JAZs (JAZ4, JAZ7, JAZ8, JAZ10, JAZ11 and JAZ13) at the stomata could be expected.

Additionally, is also shown that JAZ2 plays a major role in controlling stomatal reopening during bacterial invasion and a minor (or redundant) role in apoplastic defenses. Firstly, the dominant *JAZ2ΔJas* plants were remarkably more resistant than WT when *Pto* DC3000 was sprayed onto the leaves, but only slightly more resistant when the stomata barrier was bypassed by leaf infiltration of the bacteria. Secondly, *JAZ2ΔJas* plants did not show altered resistance to necrotrophic fungi, which further supports that its repressive effect on JA signaling does not affect apoplastic defenses. Finally, further analysis of typical JA-mediated responses such as root-growth and anthocyanin accumulation are consistent with a prevalent role of JAZ2 in stomata since only a minor effect in anthocyanin accumulation was observed in dominant *JAZ2ΔJas* and none in *JAZ2-3* loss-of-function mutants.

## Claims

1. An isolated nucleic acid sequence that comprises a modified *JAZ* nucleic acid, operably linked to a promoter that directs the expression of the nucleic acid to the guard cells of the stomata, wherein said modified *JAZ* encodes a polypeptide comprising a ZIM domain, but not a functional Jas motif; a functional variant of the modified JAZ/JAZ, a homologue or orthologue thereof.

2. The isolated nucleic acid according to claim 1 wherein the ZIM domain comprises SEQ ID NO: 27, preferably the ZIM domain comprises SEQ ID NO: 28.

3. The isolated nucleic acid according to claim 1 wherein the ZIM domain comprises the sequence SEQ ID NO: 4, preferably the ZIM domain comprises a sequence with at least 65% identity with SEQ ID NO: 5; a functional variant of the modified JAZ/JAZ, a homologue or orthologue thereof.

4. The isolated nucleic acid according to any one of claims 1 to 3 wherein the Jas motif comprises the sequence SEQ ID NO: 6, preferably comprises a sequence with at least 80% identity with SEQ ID NO: 7; a functional variant of the modified JAZ/JAZ, a homologue or orthologue thereof.

5. The isolated nucleic acid according to claim 3 which comprises a sequence with at least 70% identity with SEQ ID NO: 8 or SEQ ID NO: 9; a functional variant of the modified JAZ/JAZ, a homologue or orthologue thereof.

6. The isolated nucleic acid according to any one of claims 1 to 5 wherein the promoter comprising a nucleic acid sequence having at least 80% sequence identity to the promoter selected from the group consisting of promoter of JAZ2 and JAZ10, preferably the promoter comprising a nucleic acid sequence having at least 80% sequence identity to the promoter of JAZ2 of *A. thaliana*, more preferably the promoter is the SEQ ID NO: 11.

7. A host cell comprising the isolated nucleic acid according to any one of claims 1 to 6, or the protein encoded by the isolated nucleic acid according to any one of claims 1 to 6, wherein the cell is preferably a plant cell but not an *Arabidopsis thaliana* cell, more preferably the plant cell is a guard cell of the stomata.

8. A plant comprising the isolated nucleic acid according to any one of claims 1 to 6, or the protein encoded by the isolated nucleic acid according to any one of claims 1 to 6, or the host cell according to claim 7, wherein the plant is not an *Arabidopsis thaliana* plant.

9. A germplasm comprising the isolated nucleic acid according to any one of claims 1 to 6, or the protein encoded by the isolated nucleic acid according to any one of claims 1 to 6, or the host cell according to claim 7, wherein the germplasm is not an *Arabidopsis thaliana* germplasm.

10. A germplasm of the plant according to claim 8.

11. A use of the isolated nucleic acid according to any one of claims 1 to 6, or the host cell according to claim 7, or the germplasm according to any of claims 9 or 10, to produce a plant with resistance to biotrophic or hemi-biotrophic plant pathogens, preferably *Pseudomonas syringae,* and without modifying the level of susceptibility of said plant to necrotrophic plant pathogens, preferably *Botrytis cynerea.*

12. A method for producing a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea,* comprising:
a. transferring to the isolated plant material the isolated nucleic acid sequence as defined in any one of claims 1 to 6, wherein said transfer of said nucleic acid is performed by transformation, by gene gun, by electroporation, by viral transduction, by protoplast fusion, by a doubled haploid technique or by embryo rescue, provided that when said method involves a doubled haploid technique said method is not essentially biological,
b. identifying the plant material obtained in the step (a) comprising the modified JAZ nucleic acid operably linked to a promoter that directs the expression of the nucleic acid to the guard cells of the stomata, as defined in any of claims 1 to 6,
c. growing the plant material identified in the step (b) in a suitable medium to produce at least a plant and/or a germplasm which expresses the modified JAZ nucleic acid sequence.

13. A method for producing a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea*, comprising:
a. modifying the *JAZ* native nucleic acid of the isolated plant material to obtain the nucleic acid encoding the non-functional Jas domain as defined in any one of the claims 1 to 6 by means of the Zinc finger nuclease 1 and 2 (ZFN1 and 2) technology, TALENs or CRISP/Cas technology;
b. identifying the plant material obtained in the step (a) having the nucleic acid encoding a non-functional Jas domain,
c. growing the plant material identified in the step (b) in a suitable medium to produce at least a plant and/or a germplasm which expresses the modified *JAZ* nucleic acid sequence.

14. A method for detecting a plant with resistance to biotrophic or hemi-biotrophic plant pathogens, preferably to *Pseudomonas syringae,* and without modifying the level of susceptibility of said plant to necrotrophic plant pathogens, preferably *Botrytis cinerea,* that comprises detecting the modified *JAZ* nucleic acid operably linked to a promoter that directs the expression of the nucleic acid to the guard cells of the stomata, as defined in any of claims 1 to 6.
